(19) Europäisches Patentamt
European Patent Office
Office européen des brevets

(11) **EP 4 636 131 A2**

(12) **EUROPEAN PATENT APPLICATION**

(43) Date of publication:
**22.10.2025 Bulletin 2025/43**

(21) Application number: **25158761.4**

(22) Date of filing: **19.02.2025**

(51) International Patent Classification (IPC):
***C25B 1/02*** *(2006.01)* ***C25B 3/07*** *(2021.01)*
***C25B 15/023*** *(2021.01)* ***C25B 15/08*** *(2006.01)*

(52) Cooperative Patent Classification (CPC):
**C25B 1/02; C25B 3/07; C25B 15/023; C25B 15/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.03.2024 JP 2024044763**

(71) Applicant: **Kabushiki Kaisha Toshiba**
**Tokyo 105-0023 (JP)**

(72) Inventors:
- **ONO, Akihiko**
  **Minato-ku, Tokyo 105-0023 (JP)**
- **KUDO, Yuki**
  **Minato-ku, Tokyo 105-0023 (JP)**
- **KIYOTA, Yasuhiro**
  **Minato-ku, Tokyo 105-0023 (JP)**
- **KOFUJI, Yusuke**
  **Minato-ku, Tokyo 105-0023 (JP)**
- **KITAGAWA, Ryota**
  **Minato-ku, Tokyo 105-0023 (JP)**

(74) Representative: **Hoffmann Eitle**
**Patent- und Rechtsanwälte PartmbB**
**Arabellastraße 30**
**81925 München (DE)**

# (54) ELECTROLYSIS DEVICE AND ELECTROLYSIS METHOD

(57) An electrolysis device includes: an electrolysis cell; a cathode supply flow path; an anode supply flow path; a cathode discharge flow path; an anode discharge flow path; a cathode flow rate regulator to adjust a flow rate A of a cathode supply fluid; an anode flow rate regulator to adjust a flow rate B of a anode supply fluid; a first flowmeter to measure a flow rate C of a cathode discharge fluid; a second flowmeter to measure a flow rate D of a anode discharge fluid; and a control device to estimate a Faraday efficiency according to a relational expression for approximating the Faraday efficiency to a function including the C and D, and control the cathode flow rate regulator according to the estimated Faraday efficiency to control the A.

FIG. 1

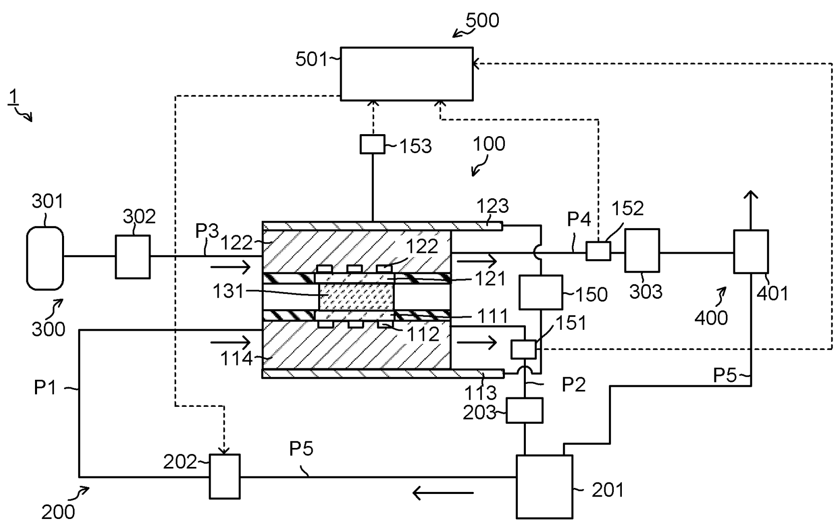


EP 4 636 131 A2

Processed by Luminess, 75001 PARIS (FR)

## Description

FIELD

Arrangements relate to an electrolysis device and an electrolysis method.

BACKGROUND

There has recently been a concern about the depletion of fossil fuels such as petroleum and coal, and expectations are increasing for sustainable renewable energy. Examples of the renewable energy include those by a solar battery and wind power generation. The amount of power generated by these depends on weather and nature conditions, and thus they have a problem of difficulty in stably supplying the power. Therefore, it has been attempted to store, in a storage battery, the power generated from the renewable energy, so as to stabilize the power. However, storing the power has problems of the cost of the storage battery and the occurrence of loss during the power storage.

In response to this point, attention has been focused on an electrolysis device that uses power generated from renewable energy to electrolyze water ($H_2O$) to produce hydrogen ($H_2$) from the water or electrochemically reduces carbon dioxide ($CO_2$) to convert it into a chemical substance (chemical energy) such as a carbon compound such as carbon monoxide ($CO$), formic acid ($HCOOH$), methanol ($CH_3OH$), methane ($CH_4$), acetic acid ($CH_3COOH$), ethanol ($C_2H_5OH$), ethane ($C_2H_6$), or ethylene ($C_2H_4$). Storing these chemical substances in a cylinder or a tank has advantages capable of reducing energy storage cost and storage loss than storing the power (electric energy) in the storage battery. An electrolysis device for reducing carbon dioxide using, for example, a silver nanoparticle catalyst for the cathode to convert carbon dioxide to carbon monoxide is under development as the electrolysis device of carbon dioxide.

RELEVANT REFERENCES

Patent Reference

Reference 1 Zengcal Liu et al., Journal of CO2 Utilization, 15, p. 50-56 (2015)
Reference 2 Sinchao Ma et al., Journal of The Electrochemical Society, 161 (10), F1124-F1131 (2014)

SUMMARY

A problem to be solved by the present invention is to prevent a decrease in electrolysis efficiency.

An electrolysis device according to an arrangement includes: an electrolysis cell having a cathode configured to reduce carbon dioxide to produce a carbon compound, an anode configured to oxidize water to produce oxygen, a cathode flow path facing on the cathode, and an anode flow path facing on the anode; a cathode supply flow path which is connected to an inlet of the cathode flow path and through which a cathode supply fluid to be supplied to the cathode flow path flows, the cathode supply fluid containing gas of the carbon dioxide; an anode supply flow path which is connected to an inlet of the anode flow path and through which an anode supply fluid to be supplied to the anode flow path flows, the anode supply fluid containing the water; a cathode discharge flow path which is connected to an outlet of the cathode flow path and through which a cathode discharge fluid to be discharged from the cathode flow path flows, the cathode discharge fluid containing the carbon compound and the carbon dioxide; an anode discharge flow path which is connected to an outlet of the anode flow path and through which an anode discharge fluid to be discharged from the anode flow path flows, the anode discharge fluid containing the oxygen and the water; a cathode flow rate regulator configured to adjust a flow rate A of the cathode supply fluid to be supplied to the cathode flow path; an anode flow rate regulator configured to adjust a flow rate B of the anode supply fluid to be supplied to the anode flow path; a first flowmeter configured to measure a flow rate C of the cathode discharge fluid to be discharged from the cathode flow path; a second flowmeter configured to measure a flow rate D of the anode discharge fluid to be discharged from the anode flow path; and a control device configured to receive a measured data of the flow rate C from the first flowmeter and a measured data of the flow rate D from the second flowmeter. The control device is configured to use the measured data of the flow rate C and the measured data of the flow rate D and estimate a value of a Faraday efficiency of the carbon compound according to a relational expression for approximating the value of the Faraday efficiency to a function including the flow rate C and the flow rate D, and control the cathode flow rate regulator according to the estimated value of the Faraday efficiency to control the flow rate A.

BRIEF DESCRIPTION OF THE DRAWINGS

FIG. 1 is a schematic view for explaining a configuration example of an electrolysis device in an arrangement.

FIG. 2 is a schematic diagram illustrating a modification example of the electrolysis device in the arrangement.

FIG. 3 is a schematic diagram illustrating a modification example of the electrolysis device of the arrangement.

FIG. 4 is a chart illustrating the relation between $FE_{CO}$ and x.

FIG. 5 is a chart illustrating the relation between $FE_{CH4}$ and x.

FIG. 6 is a chart illustrating the relation between $FE_{C2H4}$ and x.

FIG. 7 is a chart illustrating the relation between $FE_{C2H6}$ and x.

FIG. 8 is a chart illustrating the relation between $FE_{CO}$ and x.

FIG. 9 is a chart illustrating the relation between $FE_{CH4}$ and x.

FIG. 10 is a chart illustrating the relation between $FE_{C2H4}$ and x.

FIG. 11 is a chart illustrating the relation between $FE_{C2H6}$ and x.

DETAILED DESCRIPTION

Electrolysis devices in arrangements will be explained below with reference to the drawings. In the arrangements explained below, substantially the same components are denoted by the same reference signs and the explanation thereof may be partially omitted. The drawings are schematic, in which the relationship between the thickness and planar dimensions, a thickness ratio among the components, and so on may be different from actual ones.

In this specification, "connecting" may include not only directly connecting but also indirectly connecting unless otherwise specified.

FIG. 1 is a schematic view for explaining a configuration example of an electrolysis device in an arrangement. FIG. 1 illustrates an electrolysis device 1. The electrolysis device 1 includes an electrolysis part 100, an anode supply part 200, a cathode supply part 300, a cathode discharge part 400, and a control part 500.

The electrolysis part 100 includes an anode 111, an anode flow path 112, an anode current collector 113, a cathode 121, a cathode flow path 122, a cathode current collector 123, and a separator 131. The anode 111, the anode flow path 112, the cathode 121, the cathode flow path 122, and the separator 131 constitute an electrolysis cell. Examples of the electrolysis cell include a carbon dioxide electrolysis cell. The electrolysis part 100 may include a cell stack formed by stacking a plurality of electrolysis cells. The plurality of the electrolysis cells may be sandwiched between, for example, a pair of support plates and further tightened with bolts or the like.

The anode supply part 200 includes an anode collector 201, an anode flow rate regulator 202, and an anode pressure regulator 203.

The cathode supply part 300 includes a supply source 301, a cathode flow rate regulator 302, and a cathode pressure regulator 303.

The cathode discharge part 400 includes a cathode collector 401.

The control part 500 includes a control device 501.

The anode 111 is in contact with the separator 131. The anode 111 is an electrode for oxidizing an oxidizable material (substance to be oxidized) to produce an oxidation product. The anode 111 is an electrode for oxidizing, for example, water of the oxidizable material to produce oxygen ($O_2$) and hydrogen ions ($H^+$) of the oxidation product, or oxidizing hydroxide ions ($OH^-$) produced by the reduction reaction of a reducible material at the cathode 121 to produce oxygen or water.

The anode 111 preferably contains a catalyst material (anode catalyst material) capable of decreasing an overvoltage of the oxidation reaction. Examples of the catalyst material include metals such as platinum (Pt), palladium (Pd), and nickel (Ni), alloys and intermetallic compounds containing those metals, binary metal oxides such as manganese oxide (Mn-O), iridium oxide (Ir-O), nickel oxide (Ni-O), cobalt oxide (Co-O), iron oxide (Fe-O), tin oxide (Sn-O), indium oxide (In-O), ruthenium oxide (Ru-O), lithium oxide (Li-O), and lanthanum oxide (La-O), ternary metal oxides such as Ni-Co-O, Ni-Fe-O, La-CoO, Ni-La-O, and Sr-Fe-O, quaternary metal oxides such as Pb-Ru-Ir-O and La-Sr-Co-O, and metal complexes such as a Ru complex and a Fe complex.

The anode 111 includes a base material having a structure capable of moving liquid and ions between the separator 131 and the anode flow path 112, for example, a porous structure such as a mesh material, a punched material, a porous body, or a metal fiber sintered compact. The base material may be composed of a metal such as titanium (Ti), nickel (Ni), or iron (Fe) or a metal material such as an alloy (for example SUS) containing at least one of those metals, or may be composed of the aforementioned anode catalyst material. In the case of using an oxide as the anode catalyst material, it is preferable to bond or stack the anode catalyst material on the surface of the base material composed of the aforementioned metal material to form a catalyst layer. The anode catalyst material preferably has a nanoparticle, a nanostructure, a nanowire, or the like in order to enhance the oxidation reaction. The nanostructure is a structure obtained by forming nanoscale irregularities on the surface of the catalyst material.

The cathode 121 is in contact with the separator 131. The cathode 121 is an electrode (reduction electrode) for causing a reduction reaction of a reducible material (substance to be reduced) to produce a reduction product. Examples of the reducible material include carbon dioxide and so on. Examples of the reduction product include carbon compounds and ammonia. Examples of the carbon compound include carbon monoxide, formic acid (HCOOH), ethane, ethylene, methanol, acetic acid ($CH_3COOH$), ethanol, propanol ($C_3H_7OH$), and ethylene glycol ($C_2H_6O_2$). The reduction reaction at the cathode 121 may include a side reaction that causes a reduction reaction of water to produce hydrogen ($H_2$), along with the reduction reaction of the reducible material.

The cathode 121 includes a gas diffusion layer and a cathode catalyst layer provided on the gas diffusion layer. Between the gas diffusion layer and the cathode catalyst layer, a porous layer denser than the gas diffusion layer may be arranged. The gas diffusion layer is arranged on the cathode flow path 122 side, and the cathode catalyst layer is arranged on the separator 131 side. The cathode catalyst layer may enter the gas diffusion layer. The cathode catalyst layer preferably has a catalyst nanoparticle, a catalyst nanostructure, or the like. The gas diffusion layer is formed of, for example, carbon paper, carbon cloth, or the like, and may have been subjected to a water repellent treatment. The porous layer is formed of a porous body with a smaller pore size than that of the carbon paper or the carbon cloth.

By applying a moderate water repellent treatment to the gas diffusion layer, a reducible material gas reaches the cathode catalyst layer mainly by gas diffusion. The reduction reaction of the reducible material and the reduction reaction of the carbon compound produced thereby occur near the boundary between the gas diffusion layer and the cathode catalyst layer or near the cathode catalyst layer that has entered the gas diffusion layer.

The cathode catalyst layer is preferably formed of a catalyst material (cathode catalyst material) capable of decreasing an overvoltage in the reduction reaction when reducing carbon dioxide. Examples of the material include metals such as gold (Au), silver (Ag), copper (Cu), platinum (Pt), palladium (Pd), nickel (Ni), cobalt (Co), iron (Fe), manganese (Mn), titanium (Ti), cadmium (Cd), zinc (Zn), indium (In), gallium (Ga), lead (Pb), and tin (Sn), metal materials such as alloys and intermetallic compounds containing at least one of those metals, carbon materials such as carbon (C), graphene, CNT (carbon nanotube), fullerene, and ketjen black, and metal complexes such as a Ru complex and a Re complex. The cathode catalyst layer can employ various shapes such as a plate shape, a mesh shape, a wire shape, a particle shape, a porous shape, a thin film shape, and an island shape.

The cathode catalyst material forming the cathode catalyst layer preferably has a nanoparticle of the aforementioned metal material, a nanostructure of the metal material, a nanowire of the metal material, or a composite body in which the nanoparticle of the aforementioned metal material is supported on a carbon material such as a carbon particle, a carbon nanotube, or graphene. Applying the catalyst nanoparticle, catalyst nanostructure, catalyst nanowire, catalyst nano-support structure, or the like as the cathode catalyst material makes it possible to increase the reaction efficiency of the reduction reaction of the reducible material at the cathode 121.

The anode flow path 112 faces the anode 111. The anode flow path 112 has a function of allowing an anode solution containing the oxidizable material to flow therethrough and supplying the oxidizable material to the anode 111.

The anode solution is preferably a solution containing at least water ($H_2O$) of the oxidizable material. The reducible material may or may not contain a reducible material because the reducible material is supplied from the cathode flow path 122.

The anode solution may be an electrolytic solution containing an electrolyte. Examples of the electrolytic solution include aqueous solutions containing at least one selected from hydroxide ion ($OH^-$), hydrogen ion ($H^+$), potassium ion ($K^+$), sodium ion ($Na^+$), lithium ion ($Li^+$), chloride ion ($Cl^-$), bromide ion ($Br^-$), iodide ion ($I^-$), nitrate ion ($NO_3^-$), sulfate ion ($SO_4^{2-}$), phosphate ion ($PO_4^{2-}$), borate ion ($BO_3^{3-}$), and hydrogen carbonate ion ($HCO_3^-$). In order to reduce the electrical resistance of the anode solution, it is preferable to use, as liquid, an alkali solution in which an electrolyte such as potassium hydroxide or sodium hydroxide is dissolved in a high concentration. However, when the reducible material dissolves into the anode solution, the anode solution may gradually change to neutral. To continuously perform the electrolytic reaction, a large amount of alkaline solution is required in order, thus causing corrosive concerns and problems in sustainability. Therefore, when a near neutral electrolytic solution is used, a reducible material such as carbon dioxide is saturated, and an electrolytic solution having the same pH can be always used.

The anode flow path 112 is provided on a surface of a flow path plate 114. Examples of the material of the flow path plate 114 include a material having low chemical reactivity and no conductivity. Examples of such a material include insulating resin materials such as an acrylic resin, polyether ether ketone (PEEK), and a fluorocarbon resin. Note that the flow path plate 114 has screw holes for tightening which are not illustrated.

The cathode flow path 122 faces the cathode 121. The cathode flow path 122 has a function of allowing a cathode gas containing a reducible material to flow therethrough and supplying the reducible material to the cathode 121.

The cathode flow path 122 is provided on a surface of a flow path plate 124. It is preferable to use, as the material of the flow path plate 124, a material having low chemical reactivity and high conductivity. Examples of such a material include metal materials such as Ti and SUS, carbon, and so on. Note that the flow path plate 124 has an inlet and an outlet of the cathode flow path 122 and screw holes for tightening, which are not illustrated. Further, a not-illustrated packing is sandwiched at the front and the back of each of the flow path plates as necessary.

The separator 131 includes an ion exchange membrane capable of making ions move between the anode 111 and the cathode 121 and separating the anode 111 and the cathode 121 from each other. Examples of the ion exchange membrane include cation exchange membranes such as Nafion and Fremion, and anion exchange membranes such as Neosepta and Selemion. Besides the ion exchange membrane, a glass filter, a porous polymer membrane, a porous insulating material, or the like may be applied to the separator 131 as long as the material is capable of making ions move between the anode 111 and the cathode 121.

The anode 111 and the cathode 121 can be connected to a power source 150. Examples of the power source 150 are not limited to ordinary system power supplies or batteries, but may include a power source that supplies power generated by renewable energy such as solar cells or wind power generation. The power source 150 may further include a power controller that controls a voltage between the anode 111 and the cathode 121 by adjusting the output of the aforementioned power source. Note that the power source 150 may be provided outside the electrolysis device 1.

The inlet of the anode flow path 112 is connected to an anode supply flow path P1. The outlet of the anode flow path 112 is connected to an anode discharge flow path P2. The anode supply flow path P1 and the anode discharge flow path P2 are composed of a pipe, for example.

The inlet of the cathode flow path 122 is connected to a cathode supply flow path P3. The outlet of the cathode flow path 122 is connected to a cathode discharge flow path P4. The cathode supply flow path P3 and the cathode discharge flow path P4 are composed of a pipe, for example.

The temperature of the electrolysis part 100 can be measured using a temperature regulator 153 provided in the electrolysis device 1. The temperature regulator 153 may measure an exterior temperature of the electrolysis cell. The temperature regulator 153 may be provided in contact with the electrolysis cell or may be connected to the electrolysis cell.

When the reaction proceeds, the electrolysis part 100 generates heat to increase in temperature. The increase in temperature needs to be controlled to fall within a certain range so as not to deviate from the optimal operating conditions of the electrolyte membrane and the cell member. Therefore, a cooling device for cooling the electrolysis part 100 may be provided in the temperature regulator 153. The cooling device is controlled by the control part 500, for example, according to the temperature detected by the temperature regulator 153 and thereby can cool the electrolysis part 100. The temperature regulator 153 may have a heater that can heat the electrolysis part 100 by control of the control part 500 according to the detected temperature.

The anode collector 201 is connected to the anode discharge flow path P2. The anode collector 201 includes an anode tank capable of accommodating the anode fluid to be discharged from the anode flow path 112 and flowing through the anode discharge flow path P2, and an anode gas/liquid separator that separates the anode fluid into an anode waste liquid and an anode exhaust gas. The anode waste liquid contains the anode solution. The anode waste liquid is returned to the anode supply flow path P1 via a circulation flow path P5 connecting the anode supply flow path P1 and the anode discharge flow path P2, and is reused as the anode solution. The anode exhaust gas contains an oxidation product and water vapor. The anode exhaust gas may contain an unreacted oxidizable material.

The anode flow rate regulator 202 is provided in the middle of the anode supply flow path P1. The anode flow rate regulator 202 has, for example, a pump and can control the flow rate (anode inlet flow rate: flow rate B) of the anode supply fluid to be supplied to the anode flow path 112 via the anode supply flow path P1.

The anode pressure regulator 203 is provided in the middle of the anode discharge flow path P2. The anode pressure regulator 203 controls the pressure of the anode discharge flow path P2 to thereby control the pressure of the anode flow path 112.

The flow rate of the anode discharge fluid discharged from the anode flow path 112 can be measured using a flowmeter 151 provided in the electrolysis device 1. The flowmeter 151 can measure the flow rate (anode outlet flow rate: flow rate D) of the anode discharge fluid discharged from the anode flow path 112. The flowmeter 151 may be provided at a stage subsequent to the anode flow path 112 and previous to the anode pressure regulator 203. The flowmeter 151 may be provided in the middle of the anode discharge flow path P2 or may be connected to the anode discharge flow path P2.

The supply source 301 has a cylinder cabinet capable of accommodating a cathode supply fluid containing a reducible material, for example. The cathode supply fluid contains gas of carbon dioxide, for example. The cathode supply fluid may contain water vapor by humidifying the gas of carbon dioxide.

The cathode flow rate regulator 302 is provided in the middle of the cathode supply flow path P3. The cathode flow rate regulator 302 has, for example, a pump and can control the flow rate (cathode inlet flow rate: flow rate A) of the cathode supply fluid to be supplied to the cathode flow path 122.

The cathode pressure regulator 303 is provided in the middle of the cathode discharge flow path P4. The cathode pressure regulator 303 can control the pressure of the cathode discharge flow path P4 to thereby control the pressure of the cathode flow path 122.

The flow rate of the cathode discharge fluid discharged from the cathode flow path 122 can be measured using a flowmeter 152 provided in the electrolysis device 1. The flowmeter 152 can measure the flow rate (cathode outlet flow rate: flow rate C) of the cathode discharge fluid discharged from the cathode flow path 122. The flowmeter 152 may be provided at a stage subsequent to the cathode flow path 122 and previous to the cathode pressure regulator 303. The flowmeter 152 may be provided in the middle of the cathode discharge flow path P4 or may be connected to the cathode discharge flow path P4.

The cathode collector 401 is connected to the cathode discharge flow path P4. The cathode collector 401 includes a tank capable of accommodating the cathode fluid discharged from the cathode flow path 122 and flowing through the cathode discharge flow path P4, and a gas/liquid separator that separates the cathode fluid into a cathode waste liquid and a cathode exhaust gas. The cathode exhaust gas contains a reduction product, a hydrogen gas by a side reaction, and water vapor. The cathode waste liquid may contain an anode solution. The cathode waste liquid may contain an unreacted reducible material.

The cathode exhaust gas may be supplied from the cathode collector 401 to a subsequent-stage part 600 as illustrated in FIG. 2. FIG. 2 is a schematic diagram illustrating a modification example of the electrolysis device in the arrangement. The electrolysis device 1 illustrated in FIG. 2 is different from the electrolysis device 1 illustrated in FIG. 1 in that the subsequent-stage part 600 is provided, and the explanation of the electrolysis device 1 illustrated in FIG. 1 can be used as appropriate for the other parts. The subsequent-stage part 600 has a subsequent-stage device 601 and a hydrogen supply source 602.

The subsequent-stage device 601 is provided at a stage subsequent to the electrolysis device 1. The subsequent-stage device 601 can produce a compound by a chemical reaction using the carbon compound and hydrogen contained in the cathode discharge fluid. Examples of the subsequent-stage device 601 include a chemical synthesis reaction device such as a water electrolysis device and a hydrogen generator.

The control device 501 receives detection signals from, for example, the flowmeter 151, the flowmeter 152, and the temperature regulator 153, and transmits a control signal to the anode flow rate regulator 202. The control device 501 is electrically connected to the components via bidirectional signal lines whose illustration is partially omitted, and these components are collectively controlled. Note that each pipe is provided with not-illustrated valve and pump, and the opening/closing operations of the valve and pump may be controlled by signals from the control device 501. The control device 501 may be connected to the subsequent-stage device 601. The control device 501 may be connected to at least one instrument such as the power source 150, the anode pressure regulator 203, the cathode pressure regulator 303, and the temperature regulator 153.

The control device 501 may be connected to, for example, the anode pressure regulator 203, the anode flow rate regulator 202, the hydrogen supply source 602, and so on as illustrated in FIG. 3. FIG. 3 is a schematic diagram illustrating a modification example of the electrolysis device of the arrangement. For the other parts, the explanation of FIG. 2 can be used as appropriate.

The control device 501 may collect, for example, data representing at least one of an electric cell output such as the cell voltage, the cell current, the cathode potential, and the anode potential, a pressure or a pressure loss of the cathode flow path 122, and a pressure or a pressure loss of the anode flow path 112.

The control device 501 may be configured using hardware using a processor, or the like, for example. Note that each operation may be stored as an operation program on a computer-readable recording medium such as a memory, and each operation may be executed by appropriately reading the operation program stored on the recording medium by hardware.

The electrolysis device 1 may include an energy converter 701 and an energy converter 702 as illustrated in FIG. 3. FIG. 3 is a schematic diagram illustrating a modification example of the electrolysis device. For the other parts, the explanation of the electrolysis device 1 illustrated in FIG. 2 can be used as appropriate.

The energy converter 701 is provided in the middle of the anode discharge flow path P2. The energy converter 701 may be provided at a stage previous to or subsequent to the anode flow rate regulator 202. The energy converter 701 includes, for example, a gear and the like. The energy converter 701 can acquire kinetic energy from the anode discharge fluid to flow through the anode discharge flow path P2 and convert the kinetic energy to electric energy or rotational energy.

The energy converter 702 is provided in the middle of the cathode discharge flow path P4. The energy converter 702 may be provided at a stage previous to or subsequent to the cathode flow rate regulator 302. The energy converter 702 includes, for example, a gear and the like. The energy converter 702 can acquire kinetic energy from the cathode discharge fluid to flow through the cathode discharge flow path P4 and convert the kinetic energy to electric energy or rotational energy.

Next, an example of an electrolysis method using the electrolysis device 1 will be explained. The example of the electrolysis method controls the anode flow rate regulator 202 and the anode pressure regulator 203 to supply the anode supply fluid to the anode flow path 112 via the anode supply flow path P1, controls the cathode flow rate regulator 302 and the cathode pressure regulator 303 to supply the cathode supply fluid from the supply source 301 to the cathode flow path 122 via the cathode supply flow path P3, and applies voltage between the anode current collector 113 and the cathode current collector 123 from the power source 150 to supply current to the electrolysis cell via the anode 111 and the cathode 121.

When current is passed via the anode 111 and the cathode 121, the oxidation reaction occurs near the anode 111 and the reduction reaction occurs near the cathode 121 as explained below. Here, a case where carbon dioxide of the reducible material is reduced to produce carbon monoxide (CO) of the reduction product will be explained. However, the reduction product is not limited to carbon monoxide, and may also be other carbon compounds such as the aforementioned organic compounds or the like. Further, as the reaction process by the electrolysis cell, a case of producing mainly hydrogen ions ($H^+$) and a case of producing mainly hydroxide ions ($OH^-$) are considered, but the reaction process is not limited to either of these reaction processes.

The reaction process in the case of mainly oxidizing water ($H_2O$) to produce hydrogen ions ($H^+$) will be explained. When current is supplied between the anode 111 and the cathode 121, the oxidation reaction of water ($H_2O$) occurs at the anode 111 that is in contact with the anode solution flowing through the anode flow path 112. Specifically, as illustrated in Expression (1) below, $H_2O$ contained in the anode solution is oxidized to produce oxygen ($O_2$) and hydrogen ions ($H^+$).

$$2H_2O \rightarrow 4H^+ + O_2 + 4e^- \ldots \qquad (1)$$

$H^+$ produced at the anode 111 moves in the cathode gas in the cathode flow path 122 via the anode 111 and the separator 131 to reach near the cathode 121. Electrons ($e^-$) based on the current supplied to the cathode 121 from the power source 150 and $H^+$ that has moved near the cathode 121 cause the reduction reaction of carbon dioxide ($CO_2$). Specifically, as illustrated in Expression (2) below, $CO_2$ contained in the cathode gas supplied to the cathode 121 from the cathode flow path 122 is reduced to produce CO.

$$2CO_2 + 4H^+ + 4e^- \rightarrow 2CO + 2H_2O \ldots \qquad (2)$$

Next, the reaction process in the case of mainly reducing carbon dioxide ($CO_2$) to produce hydroxide ions ($OH^-$) will be explained. When current is supplied between the anode 111 and the cathode 121, water ($H_2O$) and carbon dioxide ($CO_2$) are reduced near the cathode 121 to produce carbon monoxide (CO) and hydroxide ions ($OH^-$), as illustrated in Expression (3) below. The hydroxide ions ($OH^-$) diffuse near the anode 111, and as illustrated in Expression (4) below, the hydroxide ions ($OH^-$) are oxidized to produce oxygen ($O_2$).

$$2CO_2 + 2H_2O + 4e^- \rightarrow 2CO + 4OH^- \ldots \qquad (3)$$

$$4OH^- \rightarrow 2H_2O + O_2 + 4e^- \ldots \qquad (4)$$

When the electrolysis device is operated for a long time as above, hydrogen is produced by the side reaction. Hydrogen may increase depending on the operating conditions such as the temperature of the electrolysis cell, the amount of current flowing through the electrolysis cell, the operating time, the start/stop time, and so on. Therefore, the operating conditions of the electrolysis cell need to be changed according to the change in Faraday efficiency.

Further, it is necessary to adjust the flow rate of carbon compound and the flow rate of hydrogen which are to be supplied to a device installed in the subsequent-stage device 601 according to the flow rates of the hydrogen and carbon compound to be produced by the electrolysis cell. Therefore, it is considered to measure the composition of a produced gas in order to measure a Faraday efficiency of each component, but there is such a problem that the measuring instrument is expensive and the cost of the whole system increases.

Besides, the pump for supplying fluid to the electrolysis cell requires energy for operation, and there is such a problem that the efficiency decreases as an energy conversion device.

In contrast, in the electrolysis device 1, the control device 501 estimates parameters such as a Faraday efficiency and so on using measured data of the flow rate D (anode outlet flow rate) from the flowmeter 151 and measured data of the flow rate C (cathode outlet flow rate) from the flowmeter 152, and performs a change operation of the operating conditions of the electrolysis device 1 if estimated values of the parameters do not satisfy the request criteria when operating the electrolysis device 1. Examples of the change operation of the operating conditions include control of changing the flow rate A by controlling the cathode flow rate regulator 302. By the control of the operating conditions, for example, the output of the electrolysis cell can be restored. Further, by the control of the operating conditions, an abnormality of the electrolytic reaction at the electrolysis cell can be sensed and the operation of the electrolysis cell can be stopped. Therefore, the decrease in electrolysis efficiency can be prevented.

Faraday efficiencies such as a Faraday efficiency $FE_{CO}$ of carbon monoxide and a Faraday efficiency $FE_{H2}$ of hydrogen can be normally calculated by measuring them by gas chromatography or infrared spectroscopy, or by mixing them with gas containing oxygen, burning the mixture with a catalyst such as platinum, and measuring the temperature of the resultant. Besides, Faraday efficiencies may be calculated by using the value of voltage or the value of current that is supplied to the electrolysis cell, the temperature of the electrolysis cell, the performance of gas/liquid separation between the anode flow path 112 and the cathode flow path 122, namely, the movement amount of liquid, gas, or the like between the anode flow path 112 and the cathode flow path 122, the gas amount of the product, the voltage difference with respect to the reference electrode, and parameters of them. It is possible to comprehensively predict Faraday efficiencies using these parameters, but it is difficult to perform accurate determination.

In the case of reducing carbon dioxide to produce carbon monoxide in the electrolysis part 100, it is assumed that the same molar amount of carbon dioxide as the production amount of carbon monoxide moves from the cathode flow path 122 to the anode flow path 112.

For example, in the case of producing carbon monoxide, the same molar amount of carbon dioxide moves to the anode flow path 112 by a 2-electron reaction. In the case of producing methane or ethylene, 3 moles of carbon dioxide move to the anode flow path 112 by a 6-electron reaction. In the case of producing ethane, carbon dioxide of 1/2 of the number of electrons in a reaction to reduce from 4 moles of carbon dioxide moves to the anode flow path 112 by an 8-electron reaction.

A total current value (total reaction current value) I flowing through the electrolysis cell and the cell stack in the electrolysis part 100 can be calculated by current density $\times$ electrode area $\times$ cell stack number.

The production amount (flow rate) of carbon monoxide can be calculated by total current value (total reaction current value) I(A) $\times$ 60 (s) $\times$ 96500 (c/mol)/2 (reaction electron number) $\times$ 22400 (cc/mol) $\times$ $FE_{CO}$. 60 (s) is a value for calculating coulomb from current value $\times$ time (s (sec)), and is contained for unit conversion to calculate the production amount by flow rate (ccm). 22400 (cc/mol) represents a volume of gas per mole.

The production amount (flow rate) of hydrogen can be calculated by I(A) $\times$ 60 (s) $\times$ 96500 (c/mol)/2 (reaction electron number) $\times$ 22400 (cc/mol) $\times$ (1 - $FE_{CO}$).

The amount (flow rate) of carbon dioxide moving to the anode flow path 112 can be calculated, similarly to the production amount of carbon monoxide, by I(A) $\times$ 60 (s)/96500 (c/mol)/2 (reaction electron number) $\times$ 22400 (cc/mol) $\times$ $FE_{CO}$.

The amount (flow rate) of oxygen produced by the anode 111 can be calculated by total current value (A) $\times$ 60 (s)/96500 (c/mol)/22400 (cc/mol).

The amount (flow rate) of carbon dioxide to be supplied to the cathode flow path 122 needs to be twice the amount to be converted to carbon monoxide, and can be calculated by total current value (A) $\times$ 60 (s)/96500 (c/mol)/2 (reaction electron number) $\times$ 22400 (cc/mol) $\times$ 2.

A flow rate obtained by subtracting the flow rate of carbon dioxide necessary for the conversion to carbon monoxide from the flow rate of carbon dioxide contained in the cathode supply fluid to be supplied to the cathode flow path 122 is assumed to be a flow rate a of excessive carbon dioxide.

The determination of the operation based on the flow rate C and the flow rate D can be obtained from a flow rate ratio between them. When a value obtained by dividing the flow rate D by a value obtained by subtracting the flow rate a from the flow rate C is assumed to be x, x can be calculated by x = D/(C - a).

At this time, the Faraday efficiency $FE_{CO}$ of carbon monoxide can be estimated according to a relational expression for approximating $FE_{CO}$ to a quadratic function including x as follows.

$$FE_{CO} = 14.778x^2 - 99.006x + 205.41$$

A theoretical value of the flow rate of carbon dioxide required for the reduction reaction can be calculated by total current value $\times$ 13.93, and the control device 501 controls the cathode flow rate regulator 302 to decrease the flow rate A according to the amount of the product when $FE_{CO}$ decreases. A decrease in amount of carbon dioxide in a gas component containing carbon monoxide and hydrogen is preferable for a hydrocarbon generator in the subsequent-stage device 601.

However, if the flow rate A is decreased, the reaction is brought into severe conditions, so that $FE_{CO}$ may further decrease. Hence, at the time when $FE_{CO}$ decreases, the flow rate A is further increased to bring the reaction into mild conditions, thereby making it possible to keep $FE_{CO}$.

When $FE_{CO}$ is 1.0 (100%), x is 1.33, and x increases as $FE_{CO}$ decreases as illustrated in FIG. 4. Therefore, the electrolysis cell is operated under a condition that a constant multiple of (x - 1.33) is added to the amount of carbon dioxide being the theoretical value, which is preferable to keep $FE_{CO}$. When 1 time (x - 1.33) is added, an effect is apparent, whereas when less than that is added, almost no effect is obtained. On the other hand, if more than 10 times (x - 1.33) is added, the cathode discharge fluid is diluted too much so that, for example, a process of separating carbon dioxide is needed in order to use it for the reaction in the subsequent-stage device 601 or the reaction efficiency of the subsequent-stage device 601 decreases, which is undesirable. For this reason, it is preferable that the control device 501 controls the cathode flow rate regulator 302 according to the estimated value of $FE_{CO}$ to control the flow rate A so as to satisfy an expression expressed by $I \times 13.93 + (x - 1.33) \times 1 \leq A \leq I \times 13.93 + (x - 1.33) \times 10$. 13.93 is a value calculated by 60 (s)/96500 (c/mol)/2 (reaction electron number) × 22400 (cc/mol) × 2.

This relational expression is established when the reduction product is gas of carbon monoxide of the 2-electron reaction. However, in the case where a liquid product is produced as the reduction product or a plurality of reaction products are produced at a large percentage as the reduction product, this relational expression may not be established. The reduction reaction is the case of mainly producing a gas component that is a single reduction product. Besides, this relational expression is likely to increase in error unless the Faraday efficiency of the single reduction product is 90% or more.

Next, a case of estimating a Faraday efficiency $FE_{CH4}$ in the case where the reduction product is methane of the 8-electron reaction will be explained. In the case where the reduction product is methane, a reaction based on a reaction expression expressed by $CO_2 + 8H^+ + 8e^- \rightarrow CH_4 + 2H_2O$ occurs. Eight electrons are needed for one molecule of carbon dioxide, one molecule of methane is produced, and four molecules of carbon dioxide move from the cathode flow path 122 to the anode flow path 112. In this event, $FE_{CH4}$ can be estimated according to a relational expression for approximating $FE_{CH4}$ to a quadratic function including x as follows.

$$FE_{CH4} = 12.053x^2 - 73.921x + 152.92$$

When $FE_{CH4}$ is 1.0 (100%), x is 0.83, and x increases as $FE_{CH4}$ decreases as illustrated in FIG. 5. Therefore, the electrolysis cell is operated under a condition that a constant multiple of (x - 0.83) is added to the amount of carbon dioxide being the theoretical value, which is preferable to keep $FE_{CH4}$. For this reason, it is preferable that the control device 501 controls the cathode flow rate regulator 302 according to the estimated value of $FE_{CH4}$ to control the flow rate A so as to satisfy an expression expressed by $I \times 13.93 + (x - 0.83) \times 1 \leq A \leq I \times 13.93 + (x - 0.83) \times 12$.

Though 10 times (x - 1.33) is added in the case of carbon monoxide of the 2-electron reaction, the change in Faraday efficiency and the increase rate of x in the case of methane of the 8-electron reaction are different from those of carbon monoxide. Therefore, if more than 12 times (x - 1.33) is added, the cathode discharge fluid is diluted too much, which is undesirable. This magnification is more than 10 times in the case of the 2-electron reaction, but is preferably not 10 times but up to 12 times because it is easier to separate methane and carbon dioxide from each other than to separate carbon monoxide and carbon dioxide from each other. Methane is likely to liquefy and can be easily separated by liquefaction by a low-temperature method. A magnification more than that is undesirable because the cathode discharge fluid is diluted too much so that, for example, a process of separating carbon dioxide is needed in order to use it for the reaction in the subsequent-stage device 601 or the reaction efficiency of the subsequent-stage device 601 decreases.

Next, a case of estimating a Faraday efficiency $FE_{C2H4}$ in the case where the reduction product is ethylene of the 6-electron reaction will be explained. In the case where the reduction product is ethylene, a reaction based on a reaction expression expressed by $2CO_2 + 12H^+ + 12e^- \rightarrow C_2H_4 + 4H_2O$ occurs. Six electrons are needed for one molecule of carbon dioxide, one molecule of ethylene is produced, and three molecules of carbon dioxide move from the cathode flow path 122 to the anode flow path 112. In this event, $FE_{C2H4}$ can be estimated according to a relational expression for approximating $FE_{C2H4}$ to a quadratic function including x as follows.

$$FE_{C2H4} = 12.317x^2 - 73.313x + 157.79$$

When $FE_{C2H4}$ is 1.0 (100%), x is 0.89, and x increases as $FE_{C2H4}$ decreases as illustrated in FIG. 6. Therefore, the electrolysis cell is operated under a condition that a constant multiple of (x - 0.89) is added to the amount of carbon dioxide being the theoretical value, which is preferable to keep $FE_{C2H4}$. For this reason, it is preferable that the control device 501 controls the cathode flow rate regulator 302 according to the estimated value of $FE_{C2H4}$ to control the flow rate A so as to satisfy an expression expressed by $I \times 13.93 + (x - 0.89) \times 1 \leq A \leq I \times 13.93 + (x - 0.89) \times 12$.

In the case of ethylene of the 6-electron reaction, the change in Faraday efficiency and the increase rate of x are different as compared with those of carbon monoxide. Therefore, if more than 12 times (x - 0.89) is added, the cathode discharge fluid is diluted too much, which is undesirable. The addition is preferably not 10 times but up to 12 times because it is easier to separate ethylene and carbon dioxide from each other than to separate carbon monoxide and carbon dioxide from each other. A magnification more than that is undesirable because the cathode discharge fluid is diluted so that, for example, a process of separating carbon dioxide is needed in order to use it for the reaction in the subsequent-stage device 601 or the reaction efficiency of the subsequent-stage device 601 decreases.

Next, a case of estimating a Faraday efficiency $FE_{C2H6}$ in the case where the reduction product is ethane of the 8-electron reaction will be explained. In the case where the reduction product is ethane, a reaction based on a reaction expression expressed by $2CO_2 + 16H^+ + 16e^- \rightarrow C_2H_6 + 4H_2O$ occurs. Eight electrons are needed for one molecule of carbon dioxide, one molecule of ethane is produced, and four molecules of carbon dioxide move from the cathode flow path 122 to the anode flow path 112. In this event, $FE_{C2H6}$ can be estimated according to a relational expression for approximating $FE_{C2H6}$ to a quadratic function including x as follows.

$$FE_{C2H6} = 12.053x^2 - 73.921x + 152.91$$

When $FE_{C2H6}$ is 1.0 (100%), x is 0.83, and x increases as $FE_{C2H6}$ decreases as illustrated in FIG. 7. Therefore, the electrolysis cell is operated under a condition that a constant multiple of (x - 0.83) is added to the amount of carbon dioxide being the theoretical value, which is preferable to keep $FE_{C2H6}$. For this reason, it is preferable that the control device 501 controls the cathode flow rate regulator 302 according to the estimated value of $FE_{C2H4}$ to control the flow rate A so as to satisfy an expression expressed by $I \times 13.93 + (x - 0.83) \times 1 \leq A \leq I \times 13.93 + (x - 0.83) \times 12$.

In the case of ethane of the 8-electron reaction, the change in Faraday efficiency and the increase rate of x are different as compared with those of carbon monoxide. Therefore, if more than 12 times (x - 0.83) is added, the cathode discharge fluid is diluted too much, which is undesirable. The addition is preferably not 10 times but up to 12 times because it is easier to separate ethane and carbon dioxide from each other than to separate carbon monoxide and carbon dioxide from each other. A magnification more than that is undesirable because the cathode discharge fluid is diluted so that, for example, a process of separating carbon dioxide is needed in order to use it for the reaction in the subsequent-stage device 601 or the reaction efficiency of the subsequent-stage device 601 decreases.

To more accurately measure the flow rate C and the flow rate D, it is preferable to measure the volume flow rate (substance flow rate, in other words, molar flow rate) of the substance, and it is difficult to measure the flow rate of fluid in which a carbon compound such as carbon monoxide, hydrogen, carbon dioxide, and oxygen are mixed and a ratio among the components is not decided.

The flow rate of fluid can be generally measured using a measuring instrument such a soap film flowmeter or a cylinder-type volumetric flow meter. However, there are such problems that the measuring instrument is large, the measuring instrument is expensive, an operation of the measuring instrument is complicated, automatic measurement by the measuring instrument is difficult, and so on, so that the mounting of the measuring instrument is difficult. The anode flow path 112 forms a gas/liquid two-layer flow composed of gas and the electrolytic solution, and the volume flow rate can be calculated by detecting a large amount of gas and liquid flowing through the flow path and finding its flow velocity. In this case, it is preferable to install a current meter at a stage previous to the anode collector 201 such as a trap tank which performs gas/liquid separation of the anode discharge fluid.

On the other hand, the cathode discharge fluid may contain water vapor at the same temperature as the temperature of the electrolysis cell. The cell temperature is higher than the ambient environmental temperature due to the reaction heat, and therefore water vapor in the pipe condenses and changes into liquid unless the temperature is kept. Hence, by detecting liquid flowing through the large amount of gas flowing through the pipe along the anode 111 and finding its flow velocity, the volume flow rate can be calculated. In this event, it is necessary to calculate the saturated water vapor amount from the pipe temperature and exclude the flow rate of water vapor from the flow rate of the cathode discharge fluid. In this case, it is preferable to install a current meter preceding to the cathode collector 401 having the trap tank or the like for performing gas/liquid separation of the cathode discharge fluid discharged from the cathode flow path 122.

However, it is difficult to find the volume flow rate of fluid. In contrast to this, there is a thermal mass flow meter as a flowmeter which can easily find the flow rate. The thermal mass flow meter has a thermal mass sensor used therein. The thermal mass flow meter measures the temperature of at least either the portion preceding or the portion following a heater provided in at least one of the flow path and a bypass part. Since a temperature difference occurs due to the flow velocity of fluid, the flow velocity is found from the temperature difference and the flow rate can be calculated. The thermal mass flow meter uses an element which generally changes in electrical resistance due to temperature to detect and find a resistance difference between before and after the element by a bridge circuit. In the case of bypassing, a split ratio is calculated to find the whole flow rate. However, there is such a problem in this system that the element cannot be used any longer if the gas species is different, and the thermal mass flow meter is manufactured based on nitrogen. If the gas species is different, adjustment can be made by a value of a conversion factor (CF) representing the difference in flow rate depending on the gas species, and the CF of hydrogen and the CF of carbon monoxide are 1.0 similar to the CF of nitrogen, but the CF of carbon dioxide is 0.74.

The thermal mass flow meter can perform measurement even if the gas species is different as above but has such a problem that it cannot perform measurement any longer if the fluid contains a plurality of components and the component ratio is different. The thermal mass flow meter is not applicable as it is if the component ratio of the cathode discharge fluid changes as in the carbon dioxide electrolysis cell.

The electrolysis device 1 has conditions that the carbon monoxide production amount and the substance amount of carbon dioxide moving to the anode flow path 112 are the same, and the CFs of the other products (hydrogen and oxygen) are 1.0, the gas species different in CF is only carbon dioxide, and all of the product is gas. Therefore, it is possible to use the thermal mass flow meters for the flowmeter 151 and the flowmeter 152, measure the flow rate C and the flow rate D using the thermal mass flow meters, and estimate the Faraday efficiency of the carbon compound using measured data of the flow rate C and measured data of the flow rate D.

In the case where the carbon compound is carbon monoxide, the flow rate CA of carbon dioxide contained in the cathode discharge fluid is measured by the following expression using the thermal mass flow meter based on nitrogen.

$$CA = (\text{total current value (A)} \times 60\ (s)/96500\ (c/mol)/2 \times 22400\ (cc/mol) \times FE_{CO} + a) \times 0.74$$

In the case of using the thermal mass flow meter based on nitrogen, a flow rate CB of carbon dioxide moving from the cathode flow path 122 to the anode flow path 112 is calculated by the following expression.

$$CB = (\text{total current value (A)} \times 60\ (s)/96500\ (c/mol)/2 \times 22400\ (cc/mol) \times FE_{CO} + a) \times 0.74$$

At this time, $FE_{CO}$ can be estimated according to a relational expression for approximating $FE_{CO}$ to a quadratic function including x as follows.

$$FE_{CO} = 16.857x^2 - 113.03x + 225.17$$

Even in the case of using the thermal mass flow meter as above, the Faraday efficiency of the carbon compound can be estimated. A theoretical value of carbon dioxide required for the reduction reaction can be calculated by $I \times 13.93$, and the control device 501 controls the cathode flow rate regulator 302 to increase the amount of carbon dioxide according to the amount of the carbon compound when the Faraday efficiency has decreased. This can keep the Faraday efficiency of the carbon compound. Therefore, the decrease in electrolysis efficiency can be prevented.

When $FE_{CO}$ is 1.0 (100%), x obtained by calculating the flow rate measured by the thermal mass flow meter by the CF is 1.40 and increases as $FE_{CO}$ decreases as illustrated in FIG. 8. Therefore, the electrolysis cell is operated under a condition that a constant multiple of x - 1.40 is added to the amount of carbon dioxide being the theoretical value, which is preferable to keep $FE_{CO}$. When 1 time x - 1.40 is added, an effect is apparent, whereas when less than that is added, almost no effect is obtained. On the other hand, when more than 7 times (x - 1.40) is added, the cathode discharge fluid is diluted too much so that, for example, a process of separating carbon dioxide is needed in order to use it for the reaction in the subsequent-stage device 601 or the reaction efficiency of the subsequent-stage device 601 decreases, which is undesirable. For this reason, it is preferable that the control device 501 controls the cathode flow rate regulator 302 according to the estimated value of $FE_{CO}$ to control the flow rate A so as to satisfy $I \times 13.93 + (x - 1.40) \times 1 \leq A \leq I \times 13.93 + (x - 1.40) \times 7$.

Next, a case of estimating $FE_{CH4}$ in the case where the carbon compound is methane will be explained. In the case where the carbon compound is methane, a reaction based on a reaction expression expressed by $CO_2 + 8H^+ + 8e^- \rightarrow CH_4 + 2H_2O$ occurs. Eight electrons are needed for one molecule of carbon dioxide, one molecule of methane is produced, and four molecules of carbon dioxide move from the cathode flow path 122 to the anode flow path 112. Further, the CF of methane is 0.74. In this event, $FE_{CH4}$ can be estimated according to a relational expression for approximating $FE_{CH4}$ to a quadratic function including x as follows.

$$FE_{CH4} = 46.722x^2 - 220.93x + 296.9$$

When $FE_{CH4}$ is 1.0 (100%), x is 1.19, and x increases as $FE_{CH4}$ decreases as illustrated in FIG. 9. Therefore, the electrolysis cell is operated under a condition that a constant multiple of (x - 1.19) is added to the amount of carbon dioxide being the theoretical value, which is preferable to keep $FE_{CH4}$. For this reason, it is preferable that the control device 501 controls the cathode flow rate regulator 302 according to the estimated value of $FE_{CH4}$ to control the flow rate A so as to satisfy an expression expressed by $I \times 13.93 + (x - 1.19) \times 1 \leq A \leq I \times 13.93 + (x - 1.19) \times 12$.

In the case of methane of the 8-electron reaction, the change in Faraday efficiency and the increase rate of x are different as compared with those of carbon monoxide. Therefore, if more than 12 times (x - 0.83) is added, the cathode discharge fluid is diluted too much, which is undesirable. For example, methane is likely to liquefy and can be easily separated by liquefaction by a low-temperature method. A magnification more than that is undesirable because the cathode discharge fluid is diluted so that, for example, a process of separating carbon dioxide is needed in order to use it for the reaction in the subsequent-stage device 601 or the reaction efficiency of the subsequent-stage device 601 decreases.

Next, a case of estimating $FE_{C2H4}$ in the case where the carbon compound is ethylene will be explained. In the case of ethane of the 6-electron reaction, when the carbon compound is ethylene, a reaction based on a reaction expression expressed by $2CO_2 + 12H^+ + 12e^- \rightarrow C_2H_4 + 4H_2O$ occurs. Six electrons are needed for one molecule of carbon dioxide, one molecule of ethylene is produced, and three molecules of carbon dioxide move from the cathode flow path 122 to the anode flow path 112. Further, the CF of ethylene is 0.64. In this event, $FE_{C2H4}$ can be estimated according to a relational expression for approximating $FE_{C2H4}$ to a quadratic function including x as follows.

$$FE_{C2H4} = 27.031x^2 - 167.8x + 296.12$$

When $FE_{C2H4}$ is 1.0 (100%), x is 1.56, and x increases as $FE_{C2H4}$ decreases as illustrated in FIG. 10. Therefore, the electrolysis cell is operated under a condition that a constant multiple of (x - 1.56) is added to the amount of carbon dioxide being the theoretical value, which is preferable to keep the Faraday efficiency. For this reason, it is preferable that the control device 501 controls the cathode flow rate regulator 302 according to the estimated value of $FE_{C2H4}$ to control the flow rate A so as to satisfy an expression expressed by $I \times 13.93 + (x - 1.56) \times 1) \leq A \leq I \times 13.93 + (x - 1.56) \times 8$.

In the case of ethylene of the 6-electron reaction, the change in Faraday efficiency and the increase rate of x are different as compared with those of carbon monoxide. Therefore, if more than 8 times (x - 1.56) is added, the cathode discharge fluid is diluted too much, which is undesirable. The addition is preferably not 7 times but up to 8 times because it is easier to separate ethylene and carbon dioxide from each other than to separate carbon monoxide and carbon dioxide from each other.

Next, a case of estimating the Faraday efficiency $FE_{C2H6}$ in the case where the carbon compound is ethane will be explained. In the case where the carbon compound is ethane, a reaction based on a reaction expression expressed by $2CO_2 + 16H^+ + 16e^- \rightarrow C_2H_6 + 4H_2O$ occurs. Eight electrons are needed for one molecule of carbon dioxide, one molecule of ethane is produced, and four molecules of carbon dioxide move from the cathode flow path 122 to the anode flow path 112. Further, the CF of ethane is 0.51. In this event, $FE_{C2H6}$ can be estimated according to a relational expression for approximating $FE_{C2H6}$ to a quadratic function including x as follows.

$$FE_{C2H6} = 49.875x^2 - 227.47x + 295.07$$

When $FE_{C2H6}$ is 1.0 (100%), x is 1.15, and x increases as $FE_{C2H6}$ decreases as illustrated in FIG. 11. Therefore, the electrolysis cell is operated under a condition that a constant multiple of (x - 1.15) is added to the amount of carbon dioxide being the theoretical value, which is preferable to keep $FE_{C2H6}$. It is preferable to perform control in a range from $I \times 13.93 + (x - 1.15) \times 1$ to $I \times 13.93 + (x - 1.15) \times 8$.

In the case of ethane of the 8-electron reaction, the change in Faraday efficiency and the increase rate of x are different as compared with those of carbon monoxide. Therefore, if more than 8 times (x - 1.15) is added, the cathode discharge fluid is diluted too much, which is undesirable The addition is preferably not 7 times but up to 8 times because it is easier to separate ethane and carbon dioxide from each other than to separate carbon monoxide and carbon dioxide from each other.

As explained above, it is possible to estimate the Faraday efficiency using the measured data of the flow rate C and the measured data of the flow rate D. Therefore, it is possible to determine the case, where the side reaction is mainly hydrogen and a total Faraday efficiency containing hydrogen of the main reaction and hydrogen of the side reaction is 0.8 (80%) or less, as an abnormality of the electrolytic reaction and an abnormality of the electrolysis device 1. Considerable causes of decreasing to 80% or less include the production of unexpected hydrogen and a substance other than the main product as the side reaction, the electrolytic corrosion (electric corrosion) of the anode 111 and the cathode 121, the crossover of substances between the anode flow path 112 and the cathode flow path 122, and the like. The crossover is a reaction that, for example, the produced hydrogen gas moves again to the anode flow path 112 and the moved hydrogen is oxidized at the anode 111 and is thereby converted again to water. The current consumed by this reaction leads to a decrease in total Faraday efficiency.

When the carbon compound is carbon monoxide, the amount (flow rate) of carbon monoxide to be produced can be calculated by the following expression.

$$\text{Carbon monoxide to be produced} = \text{total current value (A)} \times 60\ \text{(s)}/96500$$

(c/mol)/2 (reaction electron number) × 22400 (cc/mol) × $FE_{CO}$

Besides, the amount (flow rate) of hydrogen to be produced can be calculated by the following expression.

Amount of hydrogen to be produced = total current value (A) × 60 (s)/96500 (c/mol)/2 (reaction electron number) × 22400 (cc/mol) × (1 - $FE_{CO}$)

The flow rate C can be calculated by the expression expressed by flow rate C = flow rate A - flow rate of carbon dioxide converted to carbon monoxide + flow rate of produced carbon monoxide - flow rate of carbon dioxide moved to anode flow path 112 + flow rate of hydrogen to be produced.

Flow rate of produced carbon monoxide = flow rate of carbon dioxide moved to anode flow path 112. Flow rate of carbon dioxide converted to carbon monoxide = flow rate of produced carbon monoxide. From these relations, the flow rate C can be calculated by the expression expressed by Flow rate C = flow rate A - flow rate of carbon dioxide moved to anode flow path 112 + flow rate of produced hydrogen.

On the other hand, the flow rate D can be calculated from flow rate of carbon dioxide moved to anode flow path 112 + flow amount of produced oxygen. The flow rate of produced oxygen becomes a half of the flow rate of produced carbon monoxide.

Since flow rate of produced carbon monoxide = flow rate of carbon dioxide moved to anode flow path 112, and the flow rate of produced oxygen is a half of the flow rate of produced carbon monoxide, the flow rate D can be calculated from a value obtained by adding the half of the flow rate of produced hydrogen, namely, the flow rate of oxygen for production of hydrogen to the flow rate calculated by flow rate of carbon dioxide moved to the anode flow path 112 $\times$ 1.5. Besides, the difference between the cathode supply fluid and the cathode discharge fluid (cathode flow rate increase) can be calculated by flow rate of produced hydrogen - flow amount of carbon dioxide moved to anode flow path 112.

However, when a total Faraday efficiency $FE_{TOTAL}$ of the reduction product produced by the electrolysis cell is 80%, the flow rate CB of carbon dioxide moved to the anode flow path 112 can be calculated by the following expression.

CB = total current value (A) $\times$ 60 (s)/96500 (c/mol)/2 (reaction electron number) $\times$ 22400 (cc/mol) $\times$ $FE_{TOTAL}$ $\times$ 0.8

Besides, a flow rate HA of produced hydrogen can be calculated by the following expression. $FE_{RP}$ is a Faraday efficiency of the carbon compound.

HA = total current value (A) $\times$ 60 (s)/96500 (c/mol)/2 (reaction electron number) $\times$ 22400 (cc/mol) $\times$ (1 - $FE_{RP}$) $\times$ 0.8

Since flow rate of produced carbon monoxide = flow rate of carbon dioxide moved to anode flow path 112, the flow rate C can be calculated by the following expression.

Flow rate C = flow rate A - flow rate of carbon dioxide converted to carbon monoxide + flow rate of produced carbon monoxide - flow rate of carbon dioxide moved to anode flow path 112 + produced hydrogen amount

The flow rate increase (cathode flow rate increase Cinout) of the cathode discharge fluid with respect to the cathode supply fluid can be calculated by flow rate of produced hydrogen - flow rate of carbon dioxide moved to anode flow path 112.

Further, since 60 (s)/96500 (c/mol)/2 (reaction electron number) $\times$ 22400 (cc/mol) = 6.9637 (cc/min $\times$ c), Cinout can be calculated by the following expression.

Cinout = (total current value I(A) $\times$ 6.9637 (cc/min $\times$ c) $\times$ $FE_{H2}$) - (total current value I(A) $\times$ 6.9637 (cc/min $\times$ c) $\times$ $FE_{CO}$) = I(A) $\times$ 6.9637 (cc/min $\times$ c) $\times$ ($FE_{H2}$ - $FE_{CO}$)

On the other hand, the flow rate D can be calculated by the following expression.

D = (total current value I(A) $\times$ 6.9637 (cc/min $\times$ c) $\times$ (1/2) $\times$ ($FE_{H2}$ + $FE_{CO}$)) + (total current value I(A) $\times$ 6.9637 (cc/min $\times$ c) $\times$ $FE_{CO}$) = total current value I(A) $\times$ 6.9637 (cc/min $\times$ c) $\times$ (1/2) $\times$ ($FE_{H2}$ + (3/2 $\times$ $FE_{CO}$)))

From the above relation, $FE_{TOTAL}$ composed of a total of the Faraday efficiency of hydrogen and the Faraday efficiency of carbon monoxide can be calculated by the following expression.

$$FE_{TOTAL} = FE_{H2} + FE_{CO} = (((Cinout + A \times 2)/2 \times I(A) \times 6.9637\ (cc/min \times c)$$

$FE_{TOTAL}$ can be estimated as above using the measured data of the flow rate C and the measured data of the flow rate D by the control device 501. Therefore, for example, when $FE_{TOTAL}$ is 0.8 (80%) or less, it is possible to determine that an abnormal reaction has occurred, and perform an operation of stopping the operation of the electrolysis cell, for example, by the control device 501.

For example, the control device 501 preferably stops the operation of the electrolysis cell when the value calculated by (Cathode flow rate increase + D)/total reaction current value in the electrolysis cell/60 (s)/96500 (c/mol)/2 (reaction electron number) × 22400 (cc/mol) × 1.5

is 0.8 or less.

The calculated value corresponds to $FE_{TOTAL}$.

Next, a case of using an ordinary mass flow controller capable of measuring nitrogen and air will be considered. Since the CF of the mass flow controller for hydrogen is 1.0 and the CF of carbon dioxide is 0.74, it is only necessary to estimate the Faraday efficiency while only taking them into account.

The inlet of the electrolysis cell or the electrolysis device (system), when considered in terms of the volume flow rate, only needs to be calculated considering that the CF of carbon dioxide at an outlet of the electrolysis cell or the electrolysis device (system) is 0.74. The inlet, when considered in terms of the flow rate of the thermal mass flow controller with a CF based on nitrogen or air of 1.0, can be considered as a volume flow rate considering that the CF of carbon dioxide at the outlet is 0.74 because it is gas composed of a single component. On the other hand, an outlet gas needs to be considered for each component because it is a mixed gas of gas components different in CF.

The flow rate C can be calculated by the following expression.

Flow rate C = flow rate A - flow rate of carbon dioxide converted to carbon monoxide + flow rate of produced carbon monoxide - flow rate of carbon dioxide moved to anode flow path 112 + flow rate of produced hydrogen

Cinout can be calculated by the following expression.

Cinout = flow rate of produced hydrogen - flow rate of carbon dioxide moved to anode flow path 112

Here, the measured value by the thermal mass flow controller needs to take the CF into account regarding the decreased flow rate of gas of carbon dioxide.

Therefore, Cinout can be calculated by the following expression.

Cinout = (I(A) × 6.9637 (cc/min × c) × $FE_{H2}$) - (CF × I(A) × 6.9637 (cc/min × c) × FEco) = I(A) × 6.9637 (cc/min × c) × ($FE_{H2}$ - (CF × FEco))

On the other hand, the flow rate D is calculated. The CF of oxygen is 1.0.

The flow rate of produced oxygen can be calculated by the following expression.

Flow rate of produced oxygen = I(A) × 6.9637 (cc/min × c) × (1/2) × ($FE_{H2}$ + FEco))

The flow rate of carbon dioxide moved from the cathode flow path 122 can be calculated by the following expression.

Flow rate of carbon dioxide moved from the cathode flow path 122 = I(A) × 6.9637 (cc/min × c) × $FE_{CO}$

Further, the measured value by the thermal mass flow controller of the flow rate of carbon dioxide moved from the cathode flow path 122 can be calculated by the following expression.

Flow rate of carbon dioxide moved from the cathode flow path 122 = I(A) × 6.9637 (cc/min × c) × CF × FEco

From these relations, the flow rate D can be calculated by the following expression.

D = (total current value I(A) × 6.9637 (cc/min × c) × (1/2) × ($FE_{H2}$ + $FE_{CO}$))) + (total current value I(A) × 6.9637 (cc/min × c) × CF × $FE_{CO}$) = (I(A) × 6.9637 (cc/min × c) × ((1/2) × $FE_{H2}$ + ((1/2 + CF) × FEco)

(Measured value by thermal mass flow meter of flow rate C × 2) - (value obtained by subtracting measured value by thermal mass flow meter of flow rate C from volume flow rate of cathode supply fluid can be calculated by the following expression.

$$I(A) \times 6.9637\ (cc/min \times c) \times (1 + 3 \times CF) \times FE_{CO}$$

On the other hand, measured value by thermal mass flow meter of flow rate D - value obtained by subtracting measured value by thermal mass flow meter of flow rate C from volume flow rate (flow path A) of cathode supply fluid × (0.5 + CF) can be calculated by the following expression.

$$I(A) \times 6.9637\ (cc/min \times c) \times (2/3 + CF) \times FE_{H2})$$

From these relations, the following relational expression is established.

$FE_{TOTAL}$ = $FE_{CO}$ + $FE_{H2}$ (measured value by thermal mass flow meter of flow rate D × 2 - value obtained by subtracting measured value by thermal mass flow meter of flow rate C from volume flow rate of cathode supply fluid)/I(A) × 6.9637 (cc/min × c) × (2/3 + CF) + measured value by thermal mass flow meter of flow rate D - value obtained by subtracting measured value by thermal mass flow meter of flow rate C from volume flow rate of cathode supply fluid)/I (A) × 6.9637 (cc/min × c) × (1.5 + CF)

When the CF of carbon dioxide is 0.75, $FE_{TOTAL}$ can be calculated by the following expression.

$FE_{TOTAL}$ = (measured value by thermal mass flow meter of flow rate D × 6.22) - (value obtained by subtracting measured value by thermal mass flow meter of flow rate C from volume flow rate of cathode supply fluid × 0.0824/total reaction current value in the electrolysis cell/60 (s)/96500 (c/mol)/2 (reaction electron number) × 22400 (cc/mol) × 3.898)

$FE_{TOTAL}$ can be estimated as above using the measured data of the flow rate C and the measured data of the flow rate D by the control device 501 and, for example, when the estimated value of $FE_{TOTAL}$ is 0.8 or less, it is possible to determine that an abnormal reaction has occurred, and perform an operation of stopping the operation of the electrolysis cell by the control device 501.

In the case where the carbon compound is methane, the flow rate of the produced methane can be calculated by the following expression from the relation of flow rate of produced methane × 4 = flow rate of carbon dioxide moved to anode flow path 112.

Flow rate of produced methane = total current value I(A) × 60 (s) × 96500 (c/mol)/8 (reaction electron number) × 22400 (cc/mol) × $FE_{CH4}$

In the case where the carbon compound is ethylene, the flow rate of the produced ethylene can be calculated by the following expression because flow rate of produced ethylene × 3 = flow rate of carbon dioxide moved to anode flow path 112.

Flow rate of produced ethylene = total current value I(A) × 60 (s) × 96500 (c/mol)/6 (reaction electron number) × 22400 (cc/mol) × $FE_{C2H4}$

In the case where the carbon compound is ethane, the flow rate of the produced ethane can be calculated by the following expression from the relation of flow rate of produced ethane × 4 = flow rate of carbon dioxide moved to anode flow path 112.

Flow rate of produced ethane = I(A) × 60 (s) × 96500 (c/mol)/8 (reaction electron number) × 22400 (cc/mol) × $FE_{C2H6}$

Besides, when the reaction electron number is N, the flow rate of the produced carbon compound can be calculated by the following expression.

Flow rate of carbon compound = total current value I(A) × 60 (s) × 96500 (c/mol)/N (reaction electron number) × $FE_{RP}$

The flow rate of produced hydrogen in the 2-electron reaction can be calculated by the following expression.

Flow rate of produced hydrogen = total current value I(A) × 6.9637 (cc/min × c) × (1 - $FE_{H2}$)

In the case of methane, the flow rate C can be calculated by the following expression.

Flow rate C = flow rate A - flow rate of carbon dioxide converted to methane + flow rate of produced methane - flow rate of carbon dioxide moved to anode flow path **112** + flow rate of produced hydrogen

Further, flow rate of produced methane × 4 = amount of carbon dioxide moved to anode flow path 112, and flow rate of carbon dioxide converted to methane = flow rate of produced methane.

Further, flow rate of produced ethylene × 3 = flow rate of carbon dioxide moved to anode flow path 112, and flow rate of carbon dioxide converted to ethylene = flow rate of produced ethylene.

In the case of ethylene, the flow rate C can be calculated by the following expression.

C = A - flow rate of carbon dioxide converted to ethylene + flow rate of produced ethylene - flow rate of carbon dioxide moved to anode flow path 112 + flow rate of produced hydrogen

Further, flow rate of produced ethane × 4 = flow rate of carbon dioxide moved to anode flow path 112, and flow rate of carbon dioxide converted to ethane = flow rate of produced ethane.

The flow rate C can be calculated by the following expression.

C = A - flow rate of carbon dioxide converted to methane + flow rate of produced methane - flow rate of carbon dioxide moved to anode flow path 112 + flow rate of produced hydrogen

On the other hand, in the case of methane, the flow rate D can be calculated from a sum of the flow rate of carbon dioxide moved to the anode flow path 112 and the flow amount of produced oxygen. Here, flow rate of produced methane × 4 = flow rate of carbon dioxide moved to anode flow path 112, and the reaction of the produced methane produces twice that amount of oxygen, so that the flow rate D can be calculated by flow rate of carbon dioxide moved to anode flow path 112 × 1.5. Therefore, the following relational expression is established.

Flow rate D = total current value I(A) × 13.9278 (cc/min × c)/2 (reaction electron number) × ((1/2) $FE_{H2}$ + ((1/2) (reaction electron number) $FE_{CH4}$) + (8 (reaction electron number)/2 × $FE_{CH4}$)

In the case of ethylene, flow rate of produced ethylene × 3= flow rate of carbon dioxide moved to anode flow path 112, and the reaction of produced ethylene produces three times that amount of oxygen, so that the flow rate A can be calculated by amount of carbon dioxide moved to anode flow path 112 × 2. Therefore, the following expression is established.

Flow rate D = total current value I(A) × 13.9278 (cc/min × c)/2 (reaction electron number) × ((1/2) $FE_{H2}$ + (1/2) $FE_{C2H4}$ + 6 (reaction electron number)/2 × $FE_{C2H4}$)

In the case of ethane, flow rate of produced ethane × 4 = flow rate of carbon dioxide moved to anode flow path 112, and the reaction of produced ethane produces twice that amount of oxygen, so that the flow rate D is amount of carbon dioxide moved to the anode flow path 112 × 1.5. Therefore, the following expression is established.

Flow rate D = total current value I(A) × 13.9278 (cc/min × c)/2 (reaction electron number) × ((1/2) $FE_{H2}$ + (1/2) $FE_{C2H6}$ + 8 (reaction electron number)/2 × $FE_{C2H6}$)

In the case where the reaction electron number of the carbon compound (RP) of carbon dioxide is N (N is a natural number), the flow rate D can be calculated by the following expression.

Flow rate D = total current value I(A) × 13.9278 (cc/min × c)/2 (reaction electron number) × ((1/2) $FE_{H2}$ + (1/2) $FE_{RP}$ + N (reaction electron number)/2 × $FE_{RP}$)

Cinout can be calculated by flow rate of produced hydrogen - flow amount of carbon dioxide moved to anode flow path 112. However, when $FE_{TOTAL}$ (here, a total value of the Faraday efficiency of hydrogen and the Faraday efficiency of carbon compound) becomes 0.8, the flow rate of carbon dioxide moved to the anode flow path 112 can be calculated by the following expression.

Flow rate of carbon dioxide moved to anode flow path 112 = I(A) × 60 (s)/96500 (c/mol)/N (reaction electron number) × 22400 (cc/mol) × $FE_{RP}$ × 0.8

The amount of produced hydrogen can be calculated by the following expression.

Amount of produced hydrogen = total current value I(A) × 60 (s)/96500 (c/mol)/N (reaction electron number) × 22400 (cc/mol) × (1 - $FE_{RP}$) × 0.8

Since flow rate of produced carbon compound = flow rate of carbon dioxide moved to anode flow path 112, the flow rate C can be calculated by the following expression.

C = A - flow rate of carbon dioxide converted to carbon compound + flow rate of produced carbon compound - flow rate of carbon dioxide moved to anode flow path 112 + flow rate of produced hydrogen

Cinout can be calculated by the following expression.

Cinout = total current value (A) × 13.9275/2 × $FE_{H2}$ - I(A) × 13.9275/N (reaction electron number) × $FE_{RP}$

In the case of the carbon compound, the flow rate D can be calculated by the following expression.

D = total current value (A) × 13.9275/2 × ((1/2) $FE_{H2}$ + (1/2) FE (Faraday efficiency of carbon compound) + N (reaction electron number)/2 × $FE_{RP}$

In the case of the thermal mass flow controller, Cinout can be calculated by the following expression.

Cinout = (I(A) × 13.9275/2 × $FE_{H2}$) - (I(A) × 13.9275/N (reaction electron number) × $FE_{RP}$ × CF)

In the case of the thermal mass flow controller, the flow rate D can be calculated by the following expression.

D = I(A) × 13.9275/2 × ((1/2) $FE_{H2}$ + (1/2) $FE_{RP}$ + N (reaction electron number)/2 × $FE_{RP}$ × CF

In the case of the thermal mass flow controller, the following expression is established.

D - (1/2) × Cinout= total current value (A) × 13.9275/2 × ((1/2) × $FE_{RP}$ + N (reaction electron number)/2 × $FE_{RP}$ × CF + (1/2) × N (reaction electron number) $FE_{RP}$ × CF = total current value (A) × 13.9275 × 1/4 × $FE_{RP}$ × (1 - N (reaction electron number) × CF + 1/N (reaction electron number) × CF

From this relation, $FE_{RP}$ can be calculated by the following expression.

$FE_{RP}$= 4 × (D - (1/2) × Cinout)/total current value (A) × 13.9275 × (1 - N (reaction electron number) × CF + 1/N (reaction electron number) × CF) = 4 × (D - (1/2) × Cinout)/total current value (A) × 13.9275 × α α = (1 - N (reaction electron number) × CF + 1/N (reaction electron number) × CF)

In the case of methane of the 8-electron reaction, $\alpha$ = 9.125. In the case of the thermal mass flow controller, CF = 0.74 and therefore $\alpha_{MFC}$ = 7.0125. In the case of ethylene of the 6-electron reaction, $\alpha_{MFC}$ = 7.1666. In the case of the thermal mass flow controller, CF = 0.64 and therefore $\alpha_{MFC}$ = 4.9466. In the case of ethane of the 8-electron reaction, $\alpha_{MFC}$ = 9.125. In the case of the mass flow controller, CF = 0.51 and therefore $\alpha_{MFC}$ = 5.14375.

Besides, in the case of the thermal mass flow controller, $FE_{H2}$ can be calculated by the following expression.

$FE_{H2}$ = Cinout $\times$2/total current value (A) $\times$ 13.9275 + CF/N (reaction electron number) $\times$ $FE_{RP}$

From this relation, $FE_{TOTAL}$ composed of $FE_{H2}$ and $FE_{CO}$ can be calculated by the following expression.

$FE_{TOTAL}$ = $FE_{H2}$ + $FE_{CO}$ = Cinout $\times$ 2/total current value (A) $\times$ 13.9275 + CF/N (reaction electron number) $\times$ $FE_{RP}$ + 4 $\times$ (D - (1/2) $\times$ Cinout)/total current value (A) $\times$ 13.9275 $\times$ $\alpha$

As explained above, the control device 501 can estimate $FE_{TOTAL}$ using the measured data of the flow rate C and the measured data of the flow rate D. Therefore, for example, when $FE_{TOTAL}$ is 0.8 (80%) or less, it is possible to determine that an abnormal reaction has occurred in the electrolysis device 1, and perform an operation of stopping the operation of the electrolysis cell by the control device 501.

Further, the provision of the energy converter 701 and the energy converter 702 as illustrated in FIG. 3 makes it possible to use the kinetic energy of the anode discharge fluid and the kinetic energy of the cathode discharge fluid to drive the anode flow rate regulator 202 to supply the anode supply fluid, and to drive the cathode flow rate regulator 302 to supply the cathode supply fluid. This may result in less energy consumption of the whole electrolysis device and improved efficiency. Specifically, by providing gears and the like as the energy converter 701 and the energy converter 702, kinetic energy can be obtained.

In this case, the kinetic energy may be once converted to electric energy, and the electric energy may be converted again to kinetic energy to drive the anode flow rate regulator 202 and the cathode flow rate regulator 302.

Besides, not all of the kinetic energy can be used to drive the anode flow rate regulator 202 and the cathode flow rate regulator 302, and the kinetic energy decreases by the change efficiency. The kinetic energy is decreased by a certain percentage according to this value and used for driving. In the case of using the kinetic energy for the supply of the anode supply fluid, it is possible to supply the anode supply fluid at a sufficient flow rate for reaction, so that even if the percentage changes, the change causes very little influence and no problem. When the flow rate of the anode supply fluid is decreased, even if it is stoichiometrically excessive, a decrease in performance due to a decrease in contact area of a catalyst surface by an oxygen gas produced may become a problem, which can be solved by a countermeasure such as an increase in specific surface area of the catalyst. For example, it is preferable to use one made by stacking titanium nonwoven fabric or titanium grains and solidifying the stack by sintering or the like.

On the other hand, regarding the cathode supply fluid, when the gas amount is changed, a significant influence occurs in performance. Here, when the Faraday efficiency has decreased, the flow rate A can be increased, resulting in stabilization of the Faraday efficiency. Examples of the calculation results of the flow rate A, and the flow rate C + the flow rate D when assuming that the cathode supply fluid is supplied at a flow rate, which can be calculated by flow rate of a total of flow rate C and flow rate D $\times$ 0.7, as the flow rate A are listed in Table 1.

[Table 1]

| FE | A (ccm) | C+D (ccm) |
|---|---|---|
| 100% | 10.70 | 14.49 |
| 95% | 11.05 | 14.97 |
| 90% | 11.45 | 15.51 |
| 85% | 11.85 | 16.06 |
| 80% | 12.25 | 16.59 |
| 75% | 12.60 | 17.08 |
| 70% | 13.00 | 17.62 |
| 65% | 13.40 | 18.16 |
| 60% | 13.80 | 18.70 |
| 55% | 14.20 | 19.24 |

(continued)

| FE | A (ccm) | C+D (ccm) |
|---|---|---|
| 50% | 14.60 | 19.78 |

As listed in Table 1, the flow rate A is decreased according to the sum of the flow rate C and the flow rate D when the Faraday efficiency has decreased to decrease the flow rate of carbon dioxide of the cathode discharge fluid to thereby decrease the flow rate of carbon dioxide as carbon monoxide and hydrogen gas component, which is preferable for the hydrocarbon generator used in the subsequent-stage device 601. However, if the flow rate A is decreased, the reaction conditions become severe conditions, and the reaction proceeds in a direction in which the Faraday efficiency decreases. Hence, increasing the flow rate A when the Faraday efficiency has decreased to control the reaction conditions into milder conditions makes it possible to keep the Faraday efficiency. Therefore, a decrease in electrolysis efficiency can be prevented. Note that the use of seven out of ten of the kinetic energy of the anode discharge fluid and the cathode discharge fluid for the supply of the cathode supply fluid is the result of considering it as a decrease in efficiency.

As above, the flow rate A can be controlled without measuring the Faraday efficiency using an analytical instrument such as gas chromatograph. Further, the control device 501 can estimate the Faraday efficiency using the measured data of the flow rate C and the measured data of the flow rate D. Thus, it is possible to eliminate the need for the instrument for measuring the Faraday efficiency and to eliminate the need to separately provide an instrument for controlling the flow rate A, which is preferable.

The control device 501 preferably calculates the flow rate of hydrogen to be produced by the electrolysis device 1 from the value calculated from, for example,

$$I \times 6.964 \times (100 - 14.778x^2 - 99.006x + 205.41)$$

and controls the hydrogen supply source 602 to decrease the flow rate of fluid containing hydrogen supplied from the hydrogen supply source 602 to the subsequent-stage device 601 according to the calculated flow rate of hydrogen. The above value corresponds to, for example, the estimated value of $FE_{H2}$. For example, the flow rate corresponding to the decrease from $FE_{TOTAL}$ 100% at the start of operation which has been assumed at the beginning in the hydrogen supply source 602 is decreased from the flow rate of fluid containing hydtogen to be supplied to the subsequent-stage device 601. In other words, the flow rate of hydrogen has been increased by I (total current value) $\times$ 6.964 $\times$ 100 - $FE_{RP}$, and therefore it is preferable to correct the flow rate corresponding to the increase.

EXAMPLES

(Comparative example 1, Example 1 (carbon monoxide))

The electrolysis device having the structure illustrated in FIG. 1 was assembled and its electrolysis performance was examined. A cathode, in which carbon particles supporting gold nanoparticles were coated on carbon paper having a porous layer, was used for the electrolysis cell. The cathode was fabricated by the following procedure. First, a coating solution was prepared by mixing carbon particles supporting gold nanoparticles with pure water, a Nafion solution, and ethylene glycol. An average particle diameter of the gold nanoparticles was 8.7 nm, and a supporting amount was 18.9 mass%. The coating solution was filled into an airbrush and spray-coated onto the carbon paper provided with the porous layer by using a nitrogen gas. After the coating, this was washed under running water with pure water for 30 minutes, and then immersed in a hydrogen peroxide solution to oxidize and remove organic substances such as ethylene glycol. This was cut out to a size of 2 cm $\times$ 2 cm to form the cathode 121. A coating amount of gold was estimated to be about 0.4 $mg/cm^2$ based on the amount of gold nanoparticles and carbon particles mixed in the coating solution. For the anode 111, an electrode in which $IrO_2$ nanoparticles being an anode catalyst were coated on Ti mesh was used. The $IrO_2$/Ti mesh electrode was cut out to a size of 10 cm $\times$ 10 cm to be used as the anode 111.

The catalyst area of the electrolysis cell was set to 100 $cm^2$. The depth of the anode flow path 112 and the cathode flow path 122 was set to 1.0 mm.

The flow rate of carbon dioxide to be supplied as the cathode supply fluid was set to a constant value of 11.5 ccm per cell. Carbon dioxide in an amount slightly larger than the theoretical value was supplied to operate the electrolysis cell. As the anode supply fluid, a $KHCO_3$ solution of 0.1 M was circulated at a flow rate of 0.4 ccm.

A clamping plate, an insulating plate, a current collector plate, a cell, a current collector plate, an insulating plate, and a clamping plate are stacked in order to produce a structure. A total 0.8 A current was passed through the cell at a current density of 200 $mA/cm^2$ and a cell area of 4 $cm^2$. Though the operation generates heat, the operation was performed at almost room temperature since the heat capacity and the heat dissipation amount of the cell were large.

Data of all voltages and flow rates in the reaction was acquired by the control part 500. Further, the flow rate of the cathode discharge fluid from the cathode discharge flow path was regarded as the flow rate C and the flow rate of the anode discharge fluid from the anode discharge flow path was regarded as the flow rate D. The flow rate C and the flow rate D were measured by the volume flow rate and the thermal mass flow meter based on nitrogen. For confirmation, the flow rates of the carbon monoxide and hydrogen produced by the reduction reaction of carbon dioxide and the reduction reaction of water were analyzed by the gas chromatograph (GC). Further, for confirmation, the partial current density of carbon monoxide, the partial current density of hydrogen, and the Faraday efficiency being the ratio between the total current density and the partial current density of carbon monoxide were calculated from the product amount analyzed by the control device 501.

Table 2 lists the cell voltage and the Faraday efficiency (FE(GC)) of carbon monoxide at the beginning and after 50 hours from the start of the operation. Further,

Table 2 lists a value of x(D/C - a)) in the volumetric flow meter and x(MFC) calculated from the flow rate C and the flow rate D obtained by the thermal mass flow meter. Table 2 also lists the Faraday efficiency (estimated FE, estimated FE(MFC)) of the carbon compound estimated according to the following expressions using the value of x.

FE in case of volume flow rate = $14.778x^2 - 99.006x + 205.1$

FE in case of thermal mass flow meter = $16.857x^2 - 113.03x + 225.17$

(Examples 1 (×1, ×5, ×10, ×12))

A control of adding 1 time, 5 times, 10 times, and 12 times (x - 1.33) to the theoretical flow rate was always performed and continuous operation was performed for 50 hours. Conditions and results in this case are listed in Table 2.

(Comparative examples 1 (×0.5, ×15))

A control of adding 0.5 times and 15 times (x - 1.33) to the theoretical flow rate was always performed and continuous operation was performed for 50 hours. Conditions and results in this case are listed in Table 2.

[Table 2]

| | Example 1 (initial value) | Comparative example 1 (initial value) | Comparative example 1 (×0.5) | Example 1 (×1) | Example 1 (×5) | Example 1 (×10) | Example 1 (×12) | Comparative example 1 (×15) |
|---|---|---|---|---|---|---|---|---|
| Flow rate A [ccm] | 11.50 | 11.50 | 11.14 | 11.86 | 11.98 | 11.98 | 12.14 | 16.67 |
| $CO_2$ theoretical flow rate [ccm] | 11.14 | 11.14 | 11.14 | 11.14 | 11.14 | 11.14 | 11.14 | 11.14 |
| Excessive $CO_2$ introduction flow rate [ccm] | 0.36 | 0.36 | 0 | 0.72 | 0.84 | 0.84 | 1 | 5.53 |
| Flow rate A ($N_2$_MFC) [ccm] | 8.51 | 8.51 | 8.2436 | 8.7764 | 8.8652 | 8.8652 | 8.9836 | 12.3358 |
| Flow rate B [ccm] | 1.0 | 1.0 | 1.0 | 1.0 | 1.0 | 1.0 | 1.0 | 1.0 |
| Flow rate C [ccm] | 11.82 | 12.60 | 12.52 | 12.50 | 12.26 | 12.05 | 12.20 | 17.75 |
| Flow rate C-a [ccm] | 11.46 | 12.24 | 12.52 | 11.78 | 11.42 | 11.21 | 11.20 | 12.22 |
| Flow rate C (MPC) [ccm] | 9.98 | 10.75 | 10.55 | 10.43 | 9.87 | 9.72 | 3.75 | 10.50 |
| Flow rate C-a (MFC) [ccm] | 9.71 | 10.48 | 10.55 | 9.90 | 9.25 | 9.10 | 9.01 | 6,.41 |
| Flow rate D [ccm] | 8.35 | 7.26 | 7.35 | 7.83 | 8.05 | 8.15 | 8.12 | 7.20 |
| Flow rate D (MFC) [ccm] | 6.98 | 6.15 | 6.05 | 6.54 | 6.73 | 6.80 | 6.78 | 6.04 |
| x(D/(C-a)) | 1.37 | 1.69 | 1.70 | 1.50 | 1.42 | 1.38 | 1.38 | 1.70 |
| (MFC) | 1.43 | 1.75 | 1.74 | 1.59 | 1.47 | 1.43 | 1.44 | 1.74 |
| FE (GC)[%] | 98.0 | 80.2 | 80.8 | 88.0 | 94.5 | 97.2 | 97.0 | 80.5 |
| Estimated FE [%] | 97.4 | 80.4 | 79.5 | 89.8 | 94.6 | 97.1 | 96.9 | 79.8 |
| Estimated FE (MFC) [%] | 98.0 | 79.1 | 79.3 | 87.8 | 95.7 | 98.0 | 97.5 | 79.6 |
| Cell voltage [V] | 2.42 | 2.54 | 2.56 | 2.51 | 2.48 | 2.44 | 2.44 | 2.45 |

From Table 2, it can be confirmed that Examples 1 (×1, ×5, ×10, ×12) are lower in cell voltage after 50 hours of operation and higher in Faraday efficiency than Comparative example 1 (×0.5). This can be attributed to a result of increasing the flow rate A according to a decrease in the estimated value of the Faraday efficiency. On the other hand, in Comparative example 1 (×15), the cell voltage decreased and the Faraday efficiency also decreased.

(Comparative example 2, Example 2 (ethylene))

The cathode catalyst was changed to a Cu alloy-based nanocatalyst specialized for ethylene production, and the reaction was carried out. Further, the flow rate A was set to 11.5 ccm at the beginning, x was found from the flow rate C and the flow rate D, and each Faraday efficiency was estimated according to the above expression corresponding to the case of ethylene from the value of x. Other than that are the same as those in Comparative example 1 and Example 1. Conditions and results are listed in Table 3.

(Comparative examples 2 (×0.5, ×15))

A control of adding 0.5 times and 15 times (x - 0.89) to the theoretical flow rate was always performed and continuous operation was performed for 50 hours. Conditions and results in this case are listed in Table 3.

(Example 2 (×1, ×5, ×10, ×12))

A control of adding 1 time, 5 times, 10 times, and 12 times (x - 0.89) to the theoretical flow rate was always performed and continuous operation was performed for 50 hours. Conditions and results in this case are listed in Table 3.

[Table 3]

| | Example 2 (initial value) | Comparative example 2 (initial value) | Comparative example 2 (×0.5) | Example 2 (×1) | Example 2 (×5) | Example 2 (×10) | Example 2 (×12) | Comparative example 2 (×15) |
|---|---|---|---|---|---|---|---|---|
| Flow rate A [ccm] | 11.50 | 11.50 | 11.32 | 11.42 | 12.44 | 13.64 | 14.05 | 17.22 |
| $CO_2$ theoretical flow rate [ccm] | 11.14 | 11.14 | 11.14 | 11.14 | 11.14 | 11.14 | 11.14 | 11.14 |
| Excessive $CO_2$ introduction flow rate [ccm] | 0.36 | 0.36 | 0.18 | 0.28 | 1.3 | 2.5 | 2.91 | 6.08 |
| Flow rate A ($N_2$_MFC) [ccm] | 8.51 | 8.51 | 8.3768 | 8.4508 | 9.2056 | 10.0936 | 10.397 | 12.7428 |
| Flow rate B [ccm] | 1.0 | 1.0 | 1.0 | 1.0 | 1.0 | 1.0 | 1.0 | 1.0 |
| Flow rate C [ccm] | 8.70 | 9.61 | 9.40 | 9.08 | 9.90 | 10.98 | 11.51 | 15.58 |
| Flow rate C-a [ccm] | 8.34 | 9.25 | 9.22 | 8.80 | 8.60 | 8.48 | 8.60 | 9.50 |
| Flow rate C (MFC) [ccm] | 10.93 | 11.01 | 11.02 | 11.03 | 10.98 | 10.99 | 10.91 | 11.12 |
| Flow rate C-a (MFC) [ccm] | 10.66 | 10.74 | 10.89 | 10.82 | 10.02 | 9.14 | 8.76 | 6.62 |
| Flow rate D [ccm] | 7.77 | 7.30 | 7.25 | 7.54 | 7.62 | 7.68 | 7.72 | 7.14 |
| Flow rate D (MFC) [ccm] | 6.48 | 6.10 | 6.12 | 6.32 | 6.37 | 6.40 | 6.38 | 5.98 |
| x(D/(C-a)) | 1.07 | 1.27 | 1.27 | 1.17 | 1.13 | 1.10 | 1.11 | 1.33 |
| x(MFC) | 1.69 | 1.80 | 1.80 | 1.75 | 1.72 | 1.72 | 1.71 | 1.86 |
| FE(GC)[%] | 90.0 | 81.0 | 81.2 | 85.0 | 86.9 | 88.1 | 88.2 | 78.0 |
| Estimated FE [%] | 90.0 | 80.8 | 80.6 | 85.5 | 87.3 | 88.5 | 88.0 | 78.0 |
| Estimated FE (MFC) [%] | 90.0 | 81.3 | 81.6 | 85.6 | 87.2 | 87.7 | 88.2 | 77.6 |
| Cell voltage [V] | 3.76 | 3.88 | 3.88 | 3.75 | 3.73 | 3.73 | 3.73 | 3.78 |

From Table 3, it can be found that Comparative example 2 (×0.5) is the same as Comparative example 2 (ethylene) in Faraday efficiency and cell voltage though the flow rate A is decreased as compared with Comparative example 2 (ethylene). This can be said that though the conditions were severer for the reaction, the adjustment of the flow rate A according to the estimated Faraday efficiency led to a stabler operation. However, this was the same performance as Comparative example 2 (ethylene), and it failed to obtain the effect of improving the performance of the whole cell.

From Table 3, it can be confirmed that Examples 2 (×1, ×5, ×10, ×12) are lower in cell voltage after 50 hours of operation and higher in Faraday efficiency than Comparative example 1 (×0.5). This can be attributed to a result of increasing the flow rate A according to a decrease in the estimated value of the Faraday efficiency. On the other hand, in Comparative example 2 (×15), the cell voltage decreased and the Faraday efficiency also decreased.

(Comparative example 3, Example 3 (methane))

The cathode catalyst was changed to a Cu alloy-based nanocatalyst specialized for methane production, and the reaction was carried out. Further, the flow rate A was set to 11.5 ccm at the beginning, x was found from the flow rate C and the flow rate D, and each Faraday efficiency was estimated according to the above expression corresponding to the case of methane from the value of x. Other than that were performed similarly to Comparative example 1 and Example 1. Conditions and results are listed in Table 4.

(Comparative examples 3 (×0.5, ×15))

A control of adding 0.5 times and 15 times (x - 0.83) to the theoretical flow rate was always performed and continuous operation was performed for 50 hours. Conditions and results in this case are listed in Table 4.

(Examples 3 (×1, ×5, ×10, ×12))

A control of adding 1 time, 5 times, 10 times, and 12 times (x - 0.83) to the theoretical flow rate was always performed and continuous operation was performed for 50 hours. Other than that were performed similarly to Comparative example 3. Conditions and results in this case are listed in Table 4.

[Table 4]

| | Example 3 (initial value) | Comparative example 3 (initial value) | Comparative example 3 (×0.5) | Example 3 (×1) | Example 3 (×5) | Example 3 (×10) | Example 3 (×12) | Comparative example 3 (×15) |
|---|---|---|---|---|---|---|---|---|
| Flow rate A [ccm] | 11.50 | 11.50 | 11.34 | 11.48 | 12.62 | 14.00 | 14.49 | 17.17 |
| $CO_2$ theoretical flow rate [ccm] | 11.14 | 11.14 | 11.14 | 11.14 | 11.14 | 11.14 | 11.14 | 11.14 |
| Excessive $CO_2$ introduction flow rate [ccm] | 0.36 | 0.36 | 0.20 | 0.34 | 1.48 | 2.86 | 3.35 | 6.03 |
| Flow rate A ($N_2$_MFC) [ccm] | 8.51 | 8.51 | 8.3916 | 8.4952 | 9.3388 | 10.36 | 10.7226 | 12.7058 |
| Flow rate B [ccm] | 1.0 | 1.0 | 1.0 | 1.0 | 1.0 | 1.0 | 1.0 | 1.0 |
| Flow rate C [ccm] | 8.35 | 9.40 | 9.20 | 9.04 | 9.80 | 11.08 | 11.55 | 14.98 |
| Flow rate C-a [ccm] | 7.99 | 9.04 | 9.00 | 8.70 | 8.32 | 8.22 | 8.20 | 8.95 |
| Flow rate C (MFC) [ccm] | 8.37 | 8.43 | 8.47 | 8.43 | 8.38 | 8.38 | 8.37 | 8.46 |
| Flow rate C-a (MFC) [ccm] | 8.10 | 8.16 | 8.32 | 8.18 | 7.28 | 6.26 | 5.89 | 4.00 |
| Flow rate D [ccm] | 7.81 | 7.18 | 7.18 | 7.39 | 7.55 | 7.62 | 7.63 | 7.21 |
| Flow rate D (MFC) [ccm] | 6.46 | 6.01 | 6.06 | 6.17 | 6.34 | 6.36 | 6.34 | 6.05 |
| x(D/(C-a)) | 1.02 | 1.26 | 1.25 | 1.18 | 1.10 | 1.08 | 1.07 | 1.24 |
| x(MFC) | 1.30 | 1.40 | 1.40 | 1.37 | 1.32 | 1.32 | 1.32 | 1.40 |
| FE (GC)[%] | 89.7 | 79.2 | 79.4 | 83.0 | 86.5 | 87.7 | 87.4 | 79.3 |
| Estimated FE [%] | 89.9 | 79.0 | 79.2 | 82.6 | 86.1 | 87.2 | 87.4 | 79.7 |
| Estimated FE (MFC) [%] | 89.1 | 78.9 | 79.4 | 82.3 | 86.5 | 86.9 | 86.7 | 79.3 |
| Cell voltage [V] | 3.76 | 3.95 | 4.05 | 3.92 | 3.88 | 3.85 | 3.95 | 4.02 |

From Table 4, it can be found that Comparative example 3 (×0.5) is the same as Comparative example 3 (methane) in Faraday efficiency and cell voltage though the flow rate A is decreased as compared with Comparative example 3 (methane). This can be said that though the conditions were severer for the reaction, the adjustment of the flow rate A according to the estimated Faraday efficiency led to a stabler operation. However, this was the same performance as Comparative example 3 (methane), and it failed to obtain the effect of improving the performance of the whole cell.

From Table 4, it can be confirmed that Examples 3 (×1, ×5, ×10, ×12) are lower in cell voltage after 50 hours of operation and higher in Faraday efficiency than Comparative example 1 (×0.5). This can be attributed to a result of increasing the flow rate A according to a decrease in the estimated value of the Faraday efficiency. On the other hand, in Comparative example 3 (×15), the cell voltage decreased and the Faraday efficiency also decreased.

(Comparative example 4, Example 4 (ethane))

The cathode catalyst was changed to a Cu alloy-based nanocatalyst specialized for ethane production, and the reaction was carried out. Further, the flow rate A was set to 11.5 ccm at the beginning, x was found from the flow rate C and the flow rate D, and each Faraday efficiency was estimated according to the above expression corresponding to the case of ethane from the value of x. Other than that were performed similarly to Comparative example 1 and Example 1. Conditions and results are listed in Table 5.

(Comparative examples 4 (×0.5, ×15)

The flow rate A was set to 11.5 ccm at the beginning, x was obtained from the flow rate C and the flow rate D, a control of adding 0.5 times and 15 times (x - 0.83) to the theoretical flow rate was always performed, and continuous operation was performed for 50 hours. Conditions and results in this case are listed in Table 5.

(Examples 4 (×1.0, ×5.0, ×10, ×12))

The flow rate A was set to 11.5 ccm at the beginning, x was found from the flow rate C and the flow rate D, a control of adding 1 time, 5 times, 10 times, and 12 times (x - 0.83) to the theoretical flow rate was always performed, and continuous operation was performed for 50 hours. Conditions and results in this case are listed in Table 5.

[Table 5]

| | Example 4 (initial value) | Comparative example 4 (initial value) | Comparative example 4 (×0.5) | Example 4 (×1) | Example 4 (×5) | Example 4 (×10) | Example 4 (×12) | Comparative example 4 (×15) |
|---|---|---|---|---|---|---|---|---|
| Flow rate A [ccm] | 11.50 | 11.50 | 11.36 | 11.56 | 12.52 | 13.92 | 14.29 | 17.89 |
| $CO_2$ theoretical flow rate [ccm] | 11.14 | 11.14 | 11.14 | 11.14 | 11.14 | 11.14 | 11.14 | 11.14 |
| Excessive $CO_2$ introduction flow rate [ccm] | 0.36 | 0.36 | 0.22 | 0.42 | 1.38 | 2.78 | 3.15 | 6.75 |
| Flow rate A ($N_2$_MFC) [ccm] | 8.51 | 8.51 | 8.4064 | 8.5544 | 9.2648 | 10.3008 | 10.5746 | 13.2386 |
| Flow rate B [ccm] | 1.0 | 1.0 | 1.0 | 1.0 | 1.0 | 1.0 | 1.0 | 1.0 |
| Flow rate C [ccm] | 8.51 | 9.38 | 9.25 | 9.01 | 9.70 | 11.08 | 11.48 | 15.88 |
| Flow rate C-a [ccm] | 8.15 | 9.02 | 9.03 | 8.59 | 8.32 | 8.30 | 8.33 | 9.13 |
| Flow rate C (MFC) [ccm] | 8.05 | 8.16 | 8.18 | 8.10 | 8.06 | 8.05 | 8.04 | 8.18 |
| Flow rate C-a (MFC) [ccm] | 7.78 | 7.89 | 8.02 | 7.79 | 7.04 | 5.99 | 5.71 | 3.19 |
| Flow rate D [ccm] | 7.53 | 7.15 | 7.18 | 7.37 | 7.58 | 7.62 | 7.67 | 7.13 |
| Flow rate D (MFC) [ccm] | 6.35 | 6.03 | 6.04 | 6.20 | 6.33 | 6.35 | 6.34 | 6.03 |
| x(D/(C-a)) | 1.08 | 1.26 | 1.26 | 1.17 | 1.10 | 1.09 | 1.09 | 1.28 |
| x(MFC) | 1.27 | 1.35 | 1.35 | 1.31 | 1.27 | 1.27 | 1.27 | 1.36 |
| FE (GC)[%] | 87.2 | 78.5 | 78.7 | 83.0 | 86.2 | 87.0 | 87.1 | 78.1 |
| Estimated FE [%] | 87.0 | 78.8 | 79.0 | 83.1 | 86.3 | 86.7 | 86.9 | 78.0 |
| Estimated FE (MFC) [%] | 86.9 | 78.6 | 78.5 | 83.0 | 86.3 | 86.9 | 86.8 | 78.3 |
| Cell voltage [V] | 3.88 | 4.08 | 4.15 | 3.98 | 3.92 | 3.89 | 3.95 | 4.12 |

From Table 5, it can be found that Comparative example 4 (×0.5) is the same as Comparative example 4 (ethane) in Faraday efficiency and cell voltage though the flow rate A is decreased as compared with Comparative example 4 (ethane). This can be said that though the conditions were severer for the reaction, the adjustment of the flow rate A according to the estimated Faraday efficiency led to a stabler operation. However, this was the same performance as Comparative example 4 (ethane), and it failed to obtain the effect of improving the performance of the whole cell.

From Table 5, it can be confirmed that Examples 4 (×1, ×5, ×10, ×12) are lower in cell voltage after 50 hours of operation and higher in Faraday efficiency than Comparative example 4 (×0.5). This can be attributed to a result of increasing the flow rate A according to a decrease in the estimated value of the Faraday efficiency. On the other hand, in Comparative example 4 (×15), the cell voltage decreased and the Faraday efficiency also decreased.

If the flow rate A is increased too much as in the case of 15 times, the Faraday efficiency decreases due to the causes such as drying of the catalyst surface, drying of the separator, the pressure distribution inside the cell, and the temperature distribution. Humidification or the like may be performed to prevent the drying of the separator and the catalyst layer, and a countermeasure such as heating water at the same temperature as the cell temperature to form water vapor or the like may be taken. However, to increase the purity of the produced gas, the water vapor may need to be removed after the reaction when taking the use into account. Further, since the energy for producing water vapor is large, the overall efficiency decreases. Particularly, when the flow rate becomes large, the energy for producing water vapor, the energy for pre-heating the reaction gas, and the energy for removing the water vapor of the heated cathode discharge fluid become large, and lost thermal energy is larger. In addition, even if the Faraday efficiency is kept, the produced gas concentration of the cathode discharge fluid decreases, so that a purification process thereafter is required, leading to a decrease in efficiency of the whole system. As explained above, the increase in the flow rate A accompanies not only the decrease in efficiency but also the decrease in efficiency in the whole system, and therefore an extreme increase in the flow rate is not preferable because the whole system efficiency decreases.

As explained above, it is possible to prevent a decrease in electrolysis efficiency by controlling the flow rate A according to the estimated value of the Faraday efficiency.

It should be noted that the structures of the above-described arrangements may be employed in combination, or part thereof may be modified. While certain arrangements have been described, these arrangements have been presented by way of example only, and are not intended to limit the scope of the inventions. Indeed, the novel arrangements described herein may be embodied in a variety of other forms; furthermore, various omissions, substitutions and changes in the form of the arrangements described herein may be made without departing from the spirit of the inventions. The accompanying claims and their equivalents are intended to cover such forms or modifications as would fall within the scope and spirit of the inventions.

The above-described arrangements can be summarized into the following clauses.

(Clause 1) An electrolysis device including:

an electrolysis cell having a cathode configured to reduce carbon dioxide to produce a carbon compound, an anode configured to oxidize water to produce oxygen, a cathode flow path facing on the cathode, and an anode flow path facing on the anode;

a cathode supply flow path which is connected to an inlet of the cathode flow path and through which a cathode supply fluid to be supplied to the cathode flow path flows, the cathode supply fluid containing gas of the carbon dioxide;

an anode supply flow path which is connected to an inlet of the anode flow path and through which an anode supply fluid to be supplied to the anode flow path flows, the anode supply fluid containing the water;

a cathode discharge flow path which is connected to an outlet of the cathode flow path and through which a cathode discharge fluid to be discharged from the cathode flow path flows, the cathode discharge fluid containing the carbon compound and the carbon dioxide;

an anode discharge flow path which is connected to an outlet of the anode flow path and through which an anode discharge fluid to be discharged from the anode flow path flows, the anode discharge fluid containing the oxygen and the water;

a cathode flow rate regulator configured to adjust a flow rate A of the cathode supply fluid to be supplied to the cathode flow path;

an anode flow rate regulator configured to adjust a flow rate B of the anode supply fluid to be supplied to the anode flow path;

a first flowmeter configured to measure a flow rate C of the cathode discharge fluid to be discharged from the cathode flow path;

a second flowmeter configured to measure a flow rate D of the anode discharge fluid to be discharged from the anode flow path; and

a control device configured to receive a measured data of the flow rate C from the first flowmeter and a measured data of the flow rate D from the second flowmeter, wherein

the control device is configured to

use the measured data of the flow rate C and the measured data of the flow rate D and estimate a value of a Faraday efficiency of the carbon compound according to a relational expression for approximating the value of the Faraday efficiency to a function including the flow rate C and the flow rate D, and
control the cathode flow rate regulator according to the estimated value of the Faraday efficiency to control the flow rate A.

(Clause 2) The electrolysis device according to Clause 1, wherein

the control device is configured to control the cathode flow rate regulator according to the estimated value of the Faraday efficiency to control the flow rate A so as to satisfy

$$I \times 13.93 + (x - 1.33) \times 1 \leq A \leq I \times 13.93 + (x - 1.33) \times 10$$

where I represents a total reaction current value in the electrolysis cell, x satisfies x = D/(C - a), and a represents a flow rate of a gas of the carbon dioxide in the cathode discharge fluid.

(Clause 3) The electrolysis device according to Clause 1, wherein:

the first flowmeter is a first thermal mass flow meter;
the second flowmeter is a second thermal mass flow meter; and
the control device is configured to control the cathode flow rate regulator according to the estimated value of the Faraday efficiency to control the flow rate A so as to satisfy

$$I \times 13.93 + (x - 1.40) \times 1 \leq A \leq I \times 13.93 + (x - 1.40) \times 7$$

where I represents a total reaction current value, x satisfies x = D/(C - a), and a represents a flow rate of unreduced excessive carbon dioxide in the carbon dioxide in the cathode discharge fluid.

(Clause 4) The electrolysis device according to any one of Clause 1 to Clause 3, wherein
the control device is connected to at least one instrument selected from the group consisting of a power source configured to supply current or voltage between the anode and the cathode, a cathode pressure regulator configured to control a pressure of the cathode flow path, an anode pressure regulator configured to control a pressure of the anode flow path, and a temperature regulator configured to adjust a temperature of the electrolysis cell.
(Clause 5) The electrolysis device according to Clause 1, wherein
the control device is configured to stop operation of the electrolysis cell when a value calculated by (Cathode flow rate increase + D)/total reaction current value in the electrolysis cell/60 (s)/96500 (c/mol)/2 (reaction electron number) × 22400 (cc/mol)/1.5

is 0.8 or less.
(Clause 6) The electrolysis device according to Clause 1, wherein:

the first flowmeter is a first thermal mass flow meter;
the second flowmeter is a second thermal mass flow meter; and
the control device is configured to stop operation of the electrolysis cell when a total Faraday efficiency of a reduction product by the electrolysis cell is 0.8 or less.

(Clause 7) The electrolysis device according to any one of Clause 1 to Clause 6, wherein
the control device is connected to a hydrogen supply source configured to supply a fluid containing hydrogen to a chemical synthesis reaction device following to the electrolysis device and configured to produce a compound by a chemical reaction using the carbon compound and the hydrogen.
(Clause 8) The electrolysis device according to Clause 7, wherein:

the first flowmeter is a first thermal mass flow meter;
the second flowmeter is a second thermal mass flow meter; and
the control device is configured to

calculate a flow rate of hydrogen produced by the electrolysis device from a value calculated from

$$I \times 6.964 \times (100 - 14.778x^2 - 99.006x + 205.41),$$

and
control the hydrogen supply source to decrease a flow rate of a fluid containing the hydrogen to be supplied to the chemical synthesis reaction device according to the calculated flow rate of the hydrogen.

(Clause 9) The electrolysis device according to any one of Clause 1 to Clause 8, further including:

a first energy converter provided in a middle of the anode discharge flow path and configured to convert a first energy of the anode discharge fluid to flow through the anode discharge flow path to a first electric energy or a first rotational energy, wherein
the anode flow rate regulator is configured to be driven using the first electric energy or the first rotational energy.

(Clause 10) The electrolysis device according to any one of Clause 1 to Clause 8, further including:

a second energy converter provided in a middle of the cathode discharge flow path and configured to convert a second energy of the cathode discharge fluid to flow through the cathode discharge flow path to a second electric energy or a second rotational energy, wherein
the cathode flow rate regulator is configured to be driven using the second electric energy or the second rotational energy.

(Clause 11) An electrolysis method using an electrolysis device,

the electrolysis device including:

an electrolysis cell having a cathode configured to reduce carbon dioxide to produce a carbon compound, an anode configured to oxidize water to produce oxygen, a cathode flow path facing on the cathode, and an anode flow path facing on the anode;
a cathode supply flow path which is connected to an inlet of the cathode flow path and through which a cathode supply fluid to be supplied to the cathode flow path flows, the cathode supply fluid containing a gas of the carbon dioxide;
an anode supply flow path which is connected to an inlet of the anode flow path and through which an anode supply fluid to be supplied to the anode flow path flows, the anode supply fluid containing the water;
a cathode discharge flow path which is connected to an outlet of the cathode flow path and through which a cathode discharge fluid to be discharged from the cathode flow path flows, the cathode discharge fluid containing the carbon compound and the carbon dioxide;
an anode discharge flow path which is connected to an outlet of the anode flow path and through which an anode discharge fluid to be discharged from the anode flow path flows, the anode discharge fluid containing the oxygen and the water;
a cathode flow rate regulator configured to adjust a flow rate A of the cathode supply fluid to be supplied to the cathode flow path;
an anode flow rate regulator configured to adjust a flow rate B of the anode supply fluid to be supplied to the anode flow path;
a first flowmeter configured to measure a flow rate C of the cathode discharge fluid to be discharged from the cathode flow path; and
a second flowmeter configured to measure a flow rate D of the anode discharge fluid to be discharged from the anode flow path,

the electrolysis method including:

using a measured data of the flow rate C from the first flowmeter and a measured data of the flow rate D from the second flowmeter and estimating a value of a Faraday efficiency of the carbon compound according to a relational expression for approximating the value of the Faraday efficiency to a function including the flow rate C and the flow rate D, and
controlling the cathode flow rate regulator according to the estimated value of the Faraday efficiency to control the flow rate A.

## Claims

1. An electrolysis device comprising:

an electrolysis cell having a cathode configured to reduce carbon dioxide to produce a carbon compound, an anode configured to oxidize water to produce oxygen, a cathode flow path facing on the cathode, and an anode flow path facing on the anode;
a cathode supply flow path which is connected to an inlet of the cathode flow path and through which a cathode supply fluid to be supplied to the cathode flow path flows, the cathode supply fluid containing gas of the carbon dioxide;
an anode supply flow path which is connected to an inlet of the anode flow path and through which an anode supply fluid to be supplied to the anode flow path flows, the anode supply fluid containing the water;
a cathode discharge flow path which is connected to an outlet of the cathode flow path and through which a cathode discharge fluid to be discharged from the cathode flow path flows, the cathode discharge fluid containing the carbon compound and the carbon dioxide;
an anode discharge flow path which is connected to an outlet of the anode flow path and through which an anode discharge fluid to be discharged from the anode flow path flows, the anode discharge fluid containing the oxygen and the water;
a cathode flow rate regulator configured to adjust a flow rate A of the cathode supply fluid to be supplied to the cathode flow path;
an anode flow rate regulator configured to adjust a flow rate B of the anode supply fluid to be supplied to the anode flow path;
a first flowmeter configured to measure a flow rate C of the cathode discharge fluid to be discharged from the cathode flow path;
a second flowmeter configured to measure a flow rate D of the anode discharge fluid to be discharged from the anode flow path; and
a control device configured to receive a measured data of the flow rate C from the first flowmeter and a measured data of the flow rate D from the second flowmeter, wherein
the control device is configured to

use the measured data of the flow rate C and the measured data of the flow rate D and estimate a value of a Faraday efficiency of the carbon compound according to a relational expression for approximating the value of the Faraday efficiency to a function including the flow rate C and the flow rate D, and
control the cathode flow rate regulator according to the estimated value of the Faraday efficiency to control the flow rate A.

2. The electrolysis device according to claim 1, wherein

the control device is configured to control the cathode flow rate regulator according to the estimated value of the Faraday efficiency to control the flow rate A so as to satisfy

$$I \times 13.93 + (x - 1.33) \times 1 \leq A \leq I \times 13.93 + (x - 1.33) \times 10$$

where I represents a total reaction current value in the electrolysis cell, x satisfies x = D/(C - a), and a represents a flow rate of a gas of the carbon dioxide in the cathode discharge fluid.

3. The electrolysis device according to claim 1, wherein:

the first flowmeter is a first thermal mass flow meter;
the second flowmeter is a second thermal mass flow meter; and
the control device is configured to control the cathode flow rate regulator according to the estimated value of the Faraday efficiency to control the flow rate A so as to satisfy

$$I \times 13.93 + (x - 1.40) \times 1 \leq A \leq I \times 13.93 + (x - 1.40) \times 7$$

where I represents a total reaction current value, x satisfies x = D/(C - a), and a represents a flow rate of unreduced excessive carbon dioxide in the carbon dioxide in the cathode discharge fluid.

4. The electrolysis device according to claim 1, wherein
the control device is connected to at least one instrument selected from the group consisting of a power source configured to supply current or voltage between the anode and the cathode, a cathode pressure regulator configured to control a pressure of the cathode flow path, an anode pressure regulator configured to control a pressure of the anode flow path, and a temperature regulator configured to adjust a temperature of the electrolysis cell.

5. The electrolysis device according to claim 1, wherein

the control device is configured to stop operation of the electrolysis cell when a value calculated by (Cathode flow rate increase + D)/total reaction current value in the electrolysis cell/60 (s)/96500 (c/mol)/2 (reaction electron number) × 22400 (cc/mol)/1.5

is 0.8 or less.

6. The electrolysis device according to claim 1, wherein:

the first flowmeter is a first thermal mass flow meter;
the second flowmeter is a second thermal mass flow meter; and
the control device is configured to stop operation of the electrolysis cell when a total Faraday efficiency of a reduction product by the electrolysis cell is 0.8 or less.

7. The electrolysis device according to any one of claim 1 to claim 6, wherein
the control device is connected to a hydrogen supply source configured to supply a fluid containing hydrogen to a chemical synthesis reaction device following to the electrolysis device and configured to produce a compound by a chemical reaction using the carbon compound and the hydrogen.

8. The electrolysis device according to claim 7, wherein:

the first flowmeter is a first thermal mass flow meter;
the second flowmeter is a second thermal mass flow meter; and
the control device is configured to

calculate a flow rate of hydrogen produced by the electrolysis device from a value calculated from

$$I \times 6.964 \times (100 - 14.778x^2 - 99.006x + 205.41),$$

and
control the hydrogen supply source to decrease a flow rate of a fluid containing the hydrogen to be supplied to the chemical synthesis reaction device according to the calculated flow rate of the hydrogen.

9. The electrolysis device according to any one of claim 1 to claim 6, further comprising:

a first energy converter provided in a middle of the anode discharge flow path and configured to convert a first energy of the anode discharge fluid to flow through the anode discharge flow path to a first electric energy or a first rotational energy, wherein
the anode flow rate regulator is configured to be driven using the first electric energy or the first rotational energy.

10. The electrolysis device according to any one of claim 1 to claim 6, further comprising:

a second energy converter provided in a middle of the cathode discharge flow path and configured to convert a second energy of the cathode discharge fluid to flow through the cathode discharge flow path to a second electric energy or a second rotational energy, wherein
the cathode flow rate regulator is configured to be driven using the second electric energy or the second rotational energy.

11. An electrolysis method using an electrolysis device,

the electrolysis device comprising:

an electrolysis cell having a cathode configured to reduce carbon dioxide to produce a carbon compound, an anode configured to oxidize water to produce oxygen, a cathode flow path facing on the cathode, and an anode flow path facing on the anode;
a cathode supply flow path which is connected to an inlet of the cathode flow path and through which a cathode supply fluid to be supplied to the cathode flow path flows, the cathode supply fluid containing a gas of the carbon dioxide;
an anode supply flow path which is connected to an inlet of the anode flow path and through which an anode supply fluid to be supplied to the anode flow path flows, the anode supply fluid containing the water;
a cathode discharge flow path which is connected to an outlet of the cathode flow path and through which a cathode discharge fluid to be discharged from the cathode flow path flows, the cathode discharge fluid containing the carbon compound and the carbon dioxide;
an anode discharge flow path which is connected to an outlet of the anode flow path and through which an anode discharge fluid to be discharged from the anode flow path flows, the anode discharge fluid containing the oxygen and the water;
a cathode flow rate regulator configured to adjust a flow rate A of the cathode supply fluid to be supplied to the cathode flow path;
an anode flow rate regulator configured to adjust a flow rate B of the anode supply fluid to be supplied to the anode flow path;
a first flowmeter configured to measure a flow rate C of the cathode discharge fluid to be discharged from the cathode flow path; and
a second flowmeter configured to measure a flow rate D of the anode discharge fluid to be discharged from the anode flow path,

the electrolysis method comprising:

using a measured data of the flow rate C from the first flowmeter and a measured data of the flow rate D from the second flowmeter and estimating a value of a Faraday efficiency of the carbon compound according to a relational expression for approximating the value of the Faraday efficiency to a function including the flow rate C and the flow rate D, and
controlling the cathode flow rate regulator according to the estimated value of the Faraday efficiency to control the flow rate A.

**12.** The electrolysis method according to claim 11, wherein

the cathode flow rate regulator is controlled according to the estimated value of the Faraday efficiency to control the flow rate A so as to satisfy

$$I \times 13.93 + (x - 1.33) \times 1 \leq A \leq I \times 13.93 + (x - 1.33) \times 10$$

where I represents a total reaction current value in the electrolysis cell, x satisfies x = D/(C - a), and a represents a flow rate of a gas of the carbon dioxide in the cathode discharge fluid.

**13.** The electrolysis method according to claim 11, wherein:

the first flowmeter is a first thermal mass flow meter;
the second flowmeter is a second thermal mass flow meter; and
the cathode flow rate regulator is controlled according to the estimated value of the Faraday efficiency to control the flow rate A so as to satisfy

$$I \times 13.93 + (x - 1.40) \times 1 \leq A \leq I \times 13.93 + (x - 1.40) \times 7$$

where I represents a total reaction current value, x satisfies x = D/(C - a), and a represents a flow rate of unreduced excessive carbon dioxide in the carbon dioxide in the cathode discharge fluid.

**14.** The electrolysis method according to claim 11, wherein

the electrolysis device further comprises at least one instrument selected from the group consisting of a power source configured to supply current or voltage between the anode and the cathode, a cathode pressure regulator configured to control a pressure of the cathode flow path, an anode pressure regulator configured to control a pressure of the anode flow path, and a temperature regulator configured to adjust a temperature of the electrolysis cell.

**15.** The electrolysis method according to claim 11, wherein

operation of the electrolysis cell is stopped when a value calculated by (Cathode flow rate increase + D)/total reaction current value in the electrolysis cell/60 (s)/96500 (c/mol)/2 (reaction electron number) $\times$ 22400 (cc/mol)/1.5

is 0.8 or less.

FIG. 1

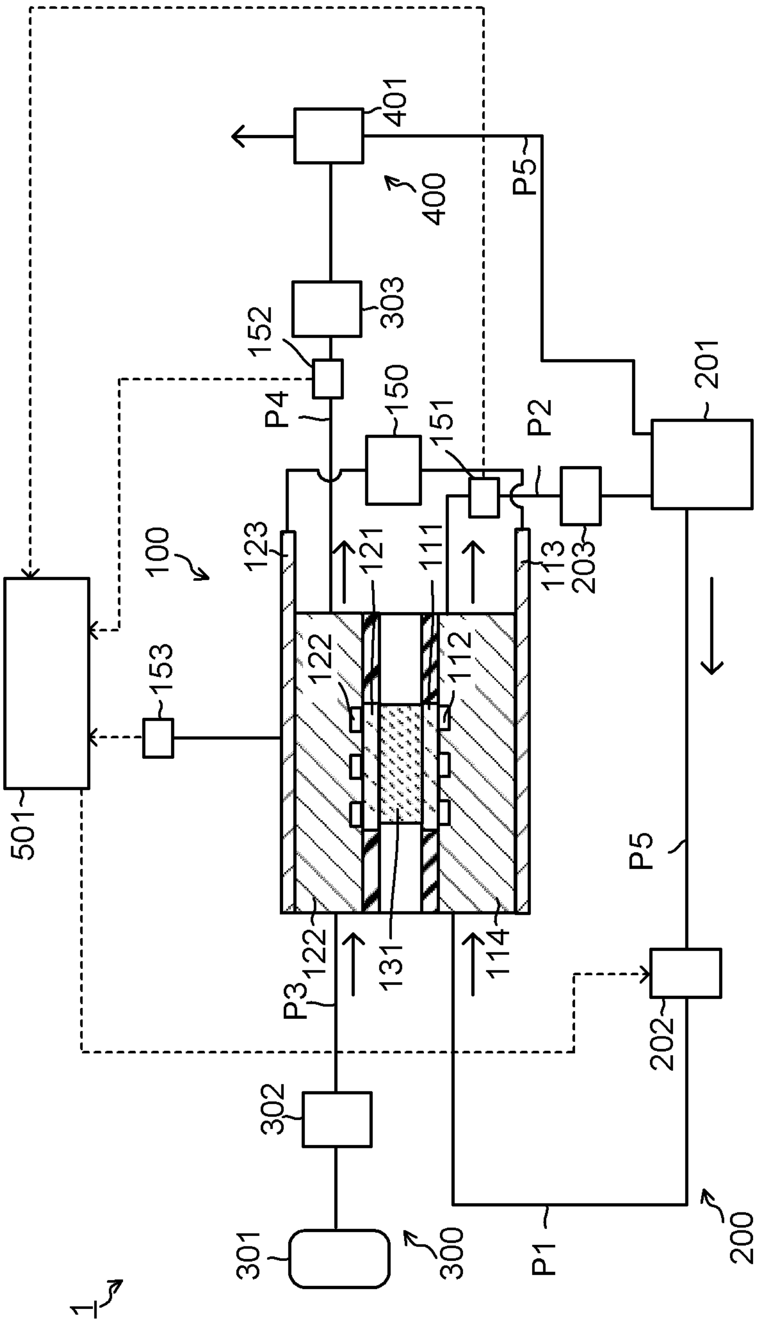
1
100
111
112
113
114
121
122
123
131
150
151
152
153
200
201
202
203
300
301
302
303
400
401
500
501
P1
P2
P3
P4
P5

FIG. 2

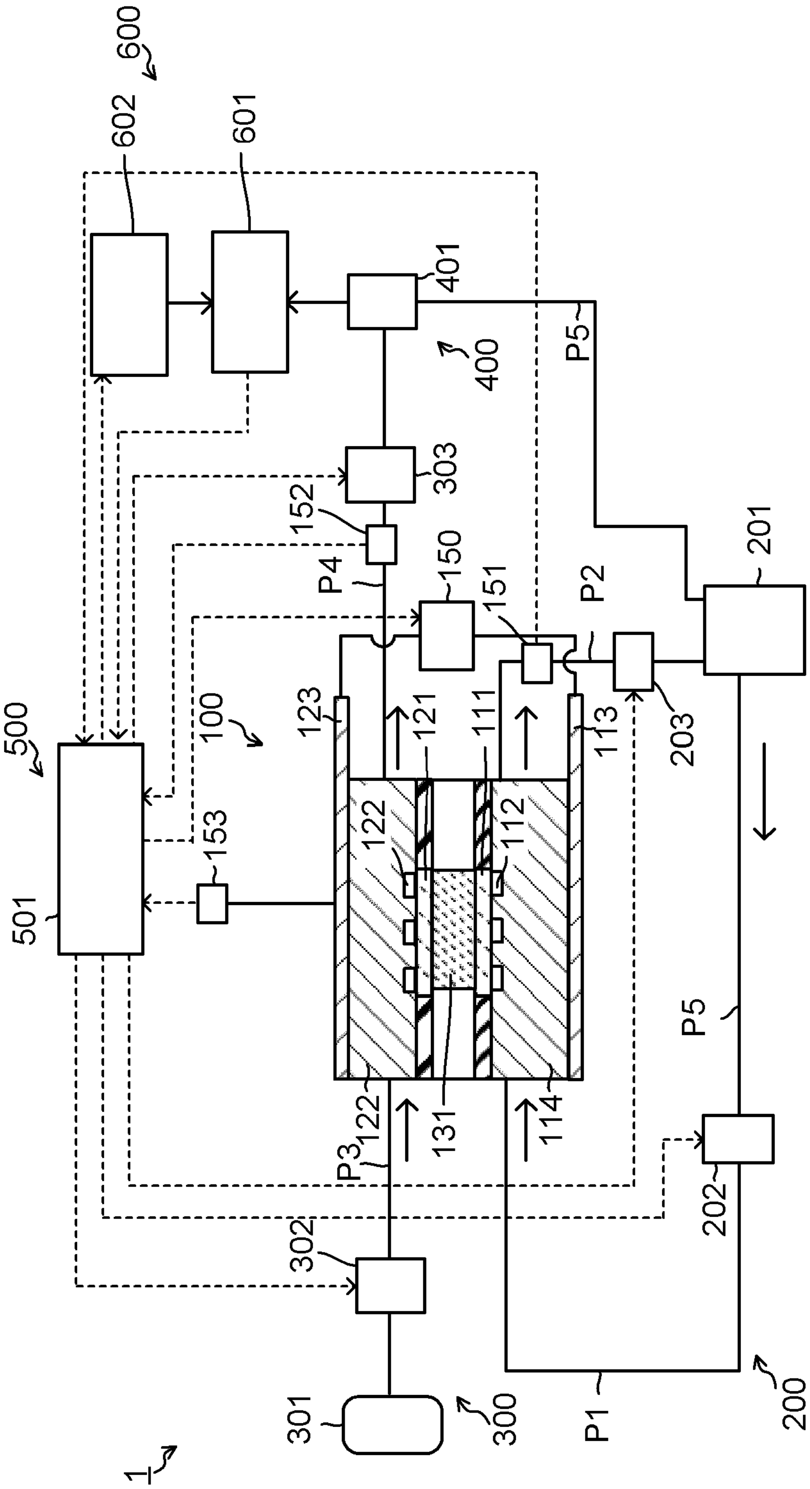
600
602
601
401
400
P5
303
152
P4
150
151
P2
201
500
100
123
121
111
113
203
122
112
153
501
P5
122
131
114
P3
302
202
301
300
P1
200
1

FIG. 3

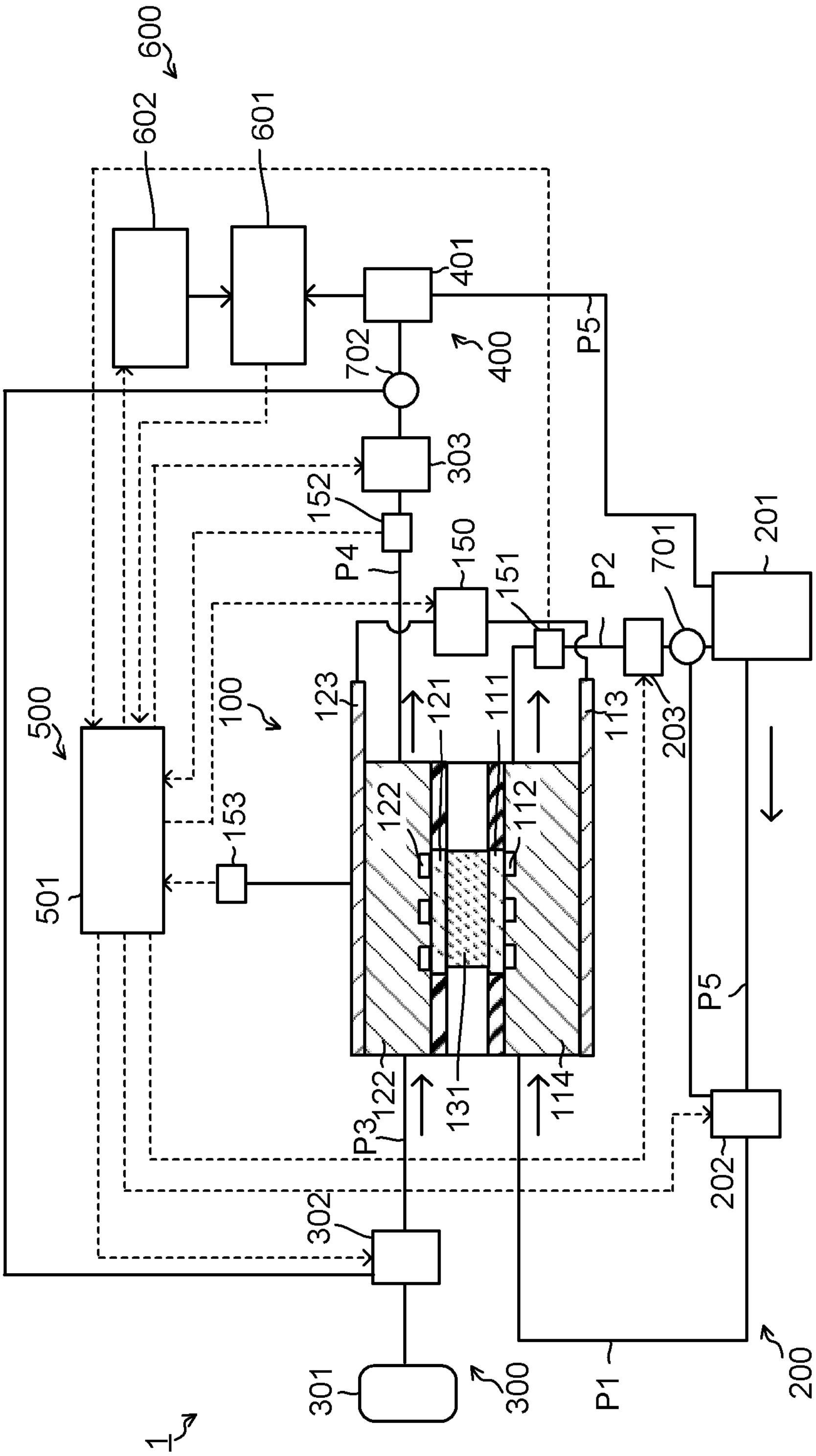
1
100
111
112
113
114
121
122
123
131
150
151
152
153
200
201
202
203
300
301
302
303
400
401
500
501
600
601
602
701
702
P1
P2
P3
P4
P5

FIG. 4

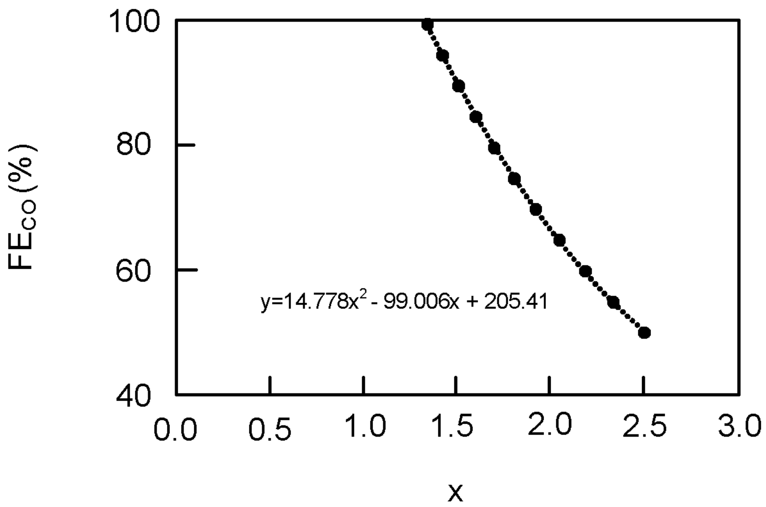
100
80
60
40
FE$_{CO}$ (%)
y=14.778x$^2$ - 99.006x + 205.41
0.0
0.5
1.0
1.5
2.0
2.5
3.0
x

FIG. 5

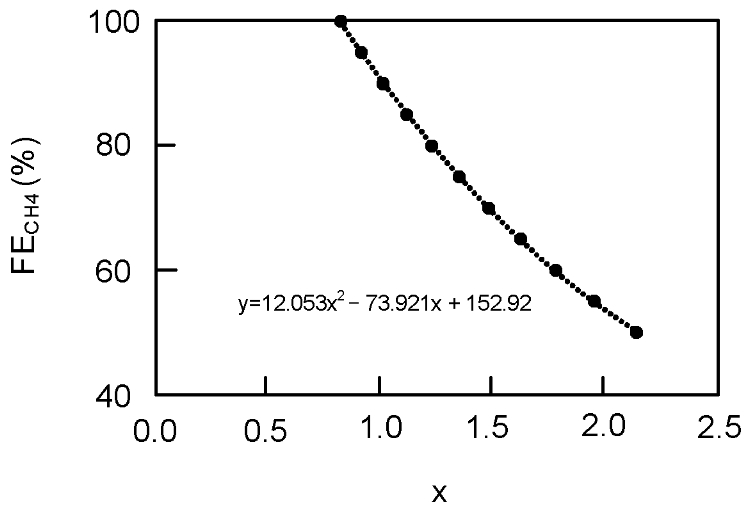
100
80
60
40
FE$_{CH4}$ (%)
y=12.053x$^2$ − 73.921x + 152.92
0.0
0.5
1.0
1.5
2.0
2.5
x

FIG. 6

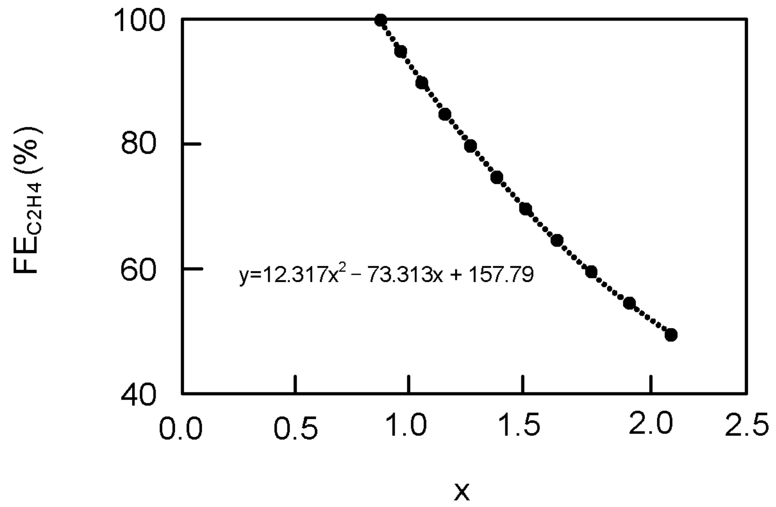
100
80
60
40
FEC2H4 (%)
y=12.317x2 − 73.313x + 157.79
0.0
0.5
1.0
1.5
2.0
2.5
x

FIG. 7

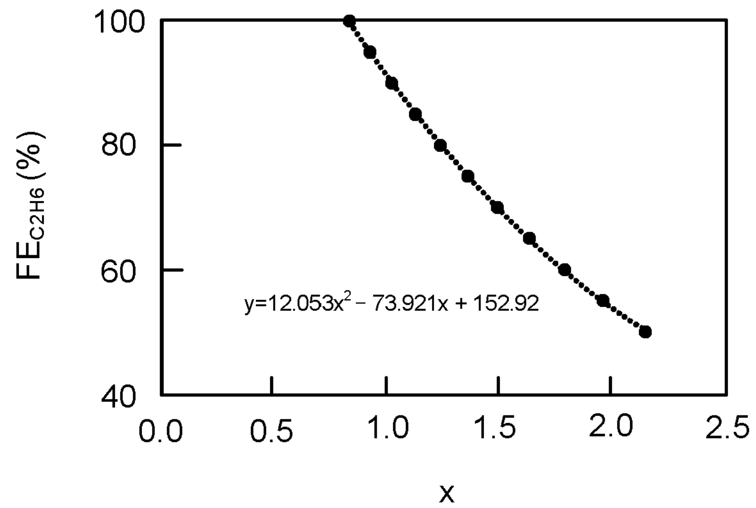
100
80
60
40
FEC2H6 (%)
y=12.053x2 − 73.921x + 152.92
0.0
0.5
1.0
1.5
2.0
2.5
x

FIG. 8

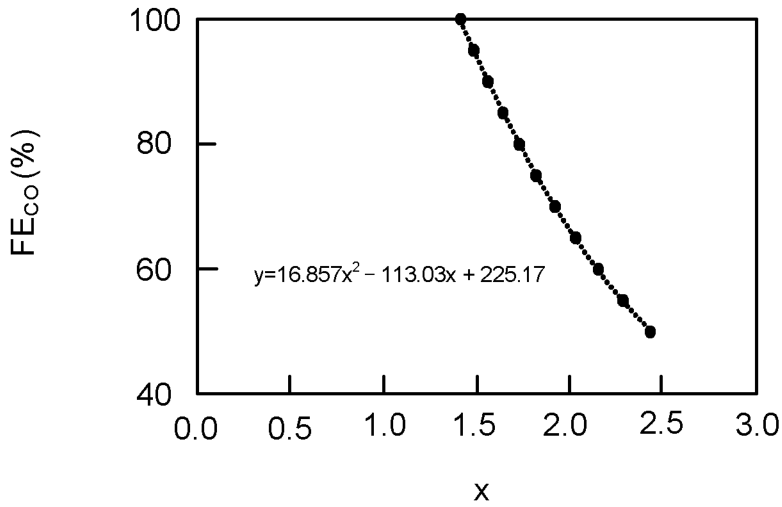
100
80
60
40
FE$_{CO}$(%)
y=16.857x$^2$ − 113.03x + 225.17
0.0
0.5
1.0
1.5
2.0
2.5
3.0
x

FIG. 9

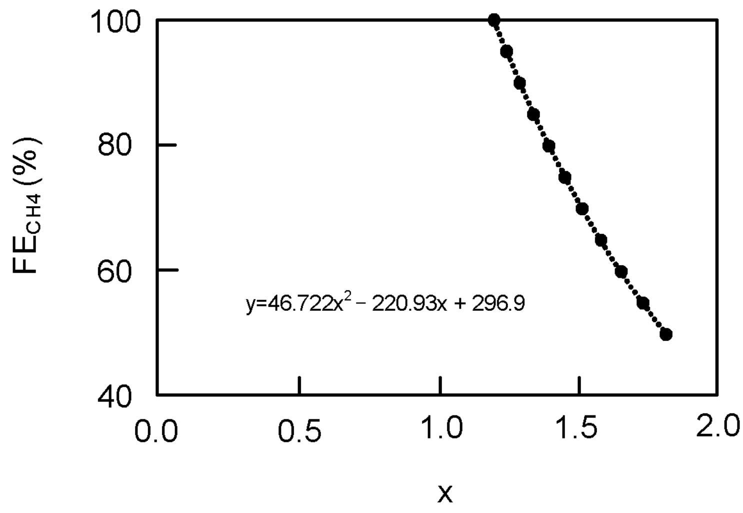
100
80
60
40
FE$_{CH4}$(%)
y=46.722x$^2$ − 220.93x + 296.9
0.0
0.5
1.0
1.5
2.0
x

FIG. 10

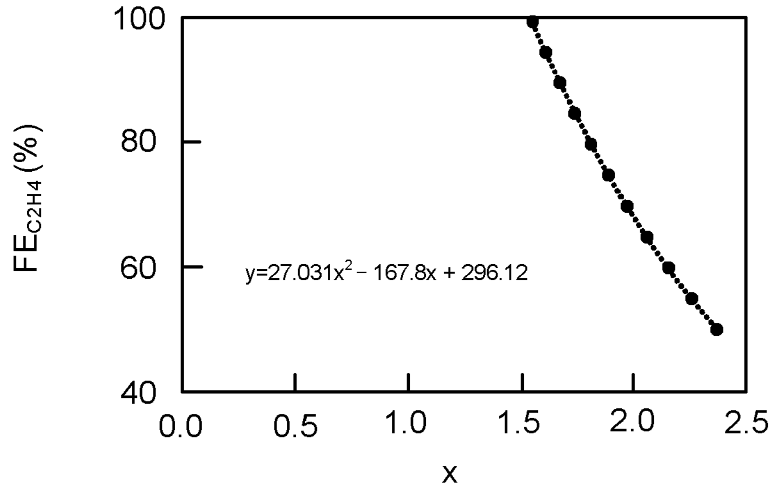
100
80
60
40
$FE_{C2H4}$ (%)
y=27.031x$^2$ − 167.8x + 296.12
0.0
0.5
1.0
1.5
2.0
2.5
x

FIG. 11

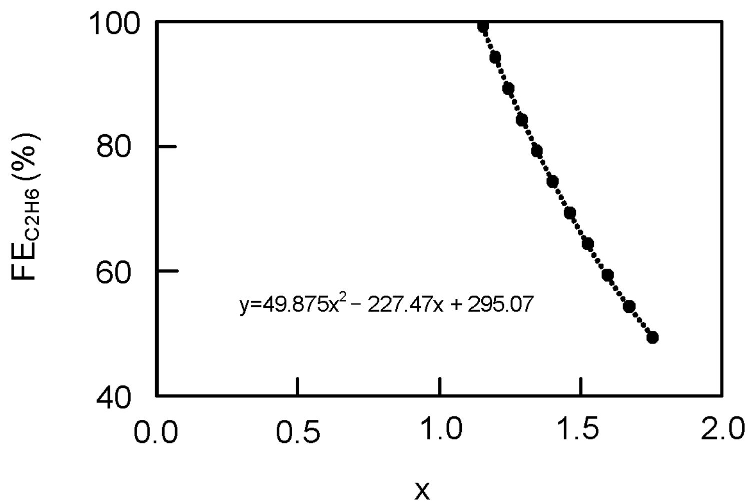
100
80
60
40
$FE_{C2H6}$ (%)
y=49.875x$^2$ − 227.47x + 295.07
0.0
0.5
1.0
1.5
2.0
x

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZENGCAL LIU et al.** *Journal of CO2 Utilization*, 2015, vol. 15, 50-56
- **SINCHAO MA et al.** *Journal of The Electrochemical Society*, 2014, vol. 161 (10), F1124-F1131